# EUROPEAN PATENT SPECIFICATION

(11) **EP 4 247 385 B1**
(45) Date of publication and mention of the grant of the patent: **19.08.2026**
(21) Application number: 21840113.1
(22) Date of filing: 18.11.2021
(51) Int. Cl.: A61K 31/52, A61N 5/10, A61K 45/06, A61K 39/395, A61P 35/00, A61P 35/04

(54) **ADMINISTRATION OF STING AGONIST, CHECKPOINT INHIBITORS, AND RADIATION**
VERABREICHUNG VON STING-AGONISTEN, CHECKPOINT-INHIBITOREN UND STRAHLUNG
ADMINISTRATION D'UN AGONISTE DE STING, D'INHIBITEURS DE POINT DE CONTRÔLE ET DE RAYONNEMENT

(30) Priority: 18.11.2020 US 202063115398 P; 21.01.2021 US 202163140125 P; 05.05.2021 US 202163184295 P; 04.11.2021 US 202163275480 P
(43) Date of publication of application: 27.09.2023
(73) Proprietor: Takeda Pharmaceutical Company Limited, Osaka-shi, Osaka 541-0045 (JP)
(72) Inventor: SHAW, Michael H., Cambridge, Massachusetts 02139 (US); SATO, Yosuke, Osaka-shi, Osaka 541-0045 (JP)
(74) Representative: Harris, Jennifer Lucy
(86) International application number: PCT/IB2021/060679
(87) International publication number: WO 2022/107027

(56) References cited:
- WO-A1-2018/118664
- WO-A1-2019/027857
- WO-A1-2020/049534
- WO-A1-2020/229982
- WO-A1-2021/005541
- WO-A2-2018/100558
- LIUFU DENG ET AL: "STING-Dependent Cytosolic DNA Sensing Promotes Radiation-Induced Type I Interferon-Dependent Antitumor Immunity in Immunogenic Tumors", IMMUNITY, vol. 41, no. 5, 1 November 2014 (2014-11-01), AMSTERDAM, NL, pages 843 - 852, XP055426473, ISSN: 1074-7613, DOI: 10.1016/j.immuni.2014.10.019

## Description

### FIELD

The present disclosure relates to methods of treating cancer. In particular, the present disclosure provides methods for treating various cancers by administering a STING (stimulator of interferon genes) agonist in combination with one or more checkpoint inhibitors and radiation.

### BACKGROUND

In 2012, there were an estimated 14 million cases of cancer diagnosed worldwide and about 8.2 million deaths. The global cancer burden is growing at an alarming pace; in 2030 alone, about 21.3 million new cancer cases and 13.1 million cancer deaths are expected to occur, simply due to the growth and aging of the population. Cancer is the second most common cause of death in the United States, exceeded only by heart disease, accounting for nearly one of every four deaths. The National Cancer Institute estimates that approximately 14.5 million Americans with a history of cancer were alive in 2014. Some of these individuals were cancer free, while others still had evidence of cancer and may have been undergoing treatment. Although medical advances have improved cancer survival rates, there is a continuing need for new and more effective treatment.

Cancer treatments have mainly relied on the surgery, radiotherapy, cytotoxic chemotherapies and combinations thereof. Within the last decade, however, targeted cancer therapies have opened a new era in the field of oncology. Targeted cancer therapies are drugs designed to interfere with specific molecules necessary for tumor growth and progression, and can include small molecules and larger chemical entities, such as monoclonal antibodies (mAbs).

STING is a transmembrane receptor localized to the ER that recognizes and binds cyclic dinucleotides. The natural ligands recognized by STING include bacteria/protozoa-derived cyclic dinucleotides (CDNs), 2',3'-cGAMP synthesized by the upstream cGAS (cyclic GMP-AMP synthase), and the like. *See* Trends in Immunology 35:88-93 (2014). It is reported that 2',3'-cGAMP, which is one of the natural ligands, is decomposed by ENPP1 (ecto-nucleotide-pyrophosphatase/phosphodiesterase), which is a pyrophosphatase/ phosphodiesterase, and that the other CDNs are decomposed by other phosphodiesterases. *See* Nat Chem Biol 10: 1043-1048 (2014); Cell Res 25:539-550 (2015*);* Biochemistry 55:837-849 (2016). STING activation by these natural ligands induces the phosphorylation of TBK1 (TANK binding kinase 1) and IRF3 (Interferon regulatory factor 3), leading to the activation of NFkB and a type-I-interferon (IFN) response, respectively. *See* Trends in Immunology 35:88-93 (2014).

The effects of STING on cancer cell growth control were demonstrated using genetically modified mice. It was reported that STING-deficient and IRF3-deficient mice show uncontrolled tumor growth, compared to wild-type mice. *See* Immunity 41: 830-842 (2014). In addition, it is also reported that the cancer cell growth in a tumor-allografted mouse was suppressed by radiation therapy, but in mice genetically deficient for STING and IFNAR1 (interferon (alpha and beta) receptor 1, receptor of type-I IFN produced by the downstream signal), the effect of the radiation therapy was reduced. *See* Immunity 41:843-852 (2014). Taking the above mentioned evidence together, STING is considered to play a critical role in suppressing cancer cell growth. Therefore, STING agonists can be used as anticancer agents. In addition, the activation of STING can further potentiate the immune effect of traditional vaccines, due to STING's ability to activate both innate and adaptive immunity. *See* Ther Adv Vaccines 1:131-143 (2013). Therefore, STING agonists can also be used as an adjuvant for various vaccines.

In addition to small molecules, targeted therapies can include monoclonal antibodies. For example, among the many known monoclonal antibody targeted therapies are monoclonal antibodies to PD-1 (e.g., nivolumab/Opdivo^{®}, and pembrolizumab/Keytruda^{®}), monoclonal antibodies to PD-L1 (e.g., atezolizumab/Tecentriq^{®}, durvalumab/Imfinzi^{®}, and avelumab/Bavencio^{®}), and monoclonal antibodies to CTLA-4 (e.g., ipilimumab/Yervoy^{®}). Thus, some cancers may be PD-1-mediated disorders, PD-L1-mediated disorders, and CTLA-4-mediated disorders. Additional monoclonal antibody targeted therapies include, but are not limited to, monoclonal antibodies to CD20 (e.g. rituximab/Rituxan^{®}) CD52 (e.g., alemtuzumab/Campath^{®}), VEGF (e.g., bevacizumab/Avastin^{®}), HER2 (e.g., trastuzumab/Herceptin^{®} for treating Her2+ breast and stomach cancers), and EGFR (e.g., cetuximab/Erbitux^{®} for treating colorectal cancer).

Another therapy can include radiation or radiotherapy. Radiotherapy is a pillar of cancer treatment with approximately 50% of cancer patients receiving radiotherapy at some point in the course of their disease. Cancer, 104(6):1129-37 (2005). In the setting of metastatic disease from solid tumors - including Non-Small-Cell Lung Cancer (NSCLC), Triple Negative Breast Cancer (TNBC) and Squamous Cell Carcinoma of Head and Neck (SCCHN) - radiotherapy is often used with palliative intent. Technological advances have allowed radiotherapy to be delivered more precisely with techniques such as three dimensional conformal radiotherapy, image-guided intensity modulated radiotherapy, and stereotactic ablative radiotherapy (SAbR). BMJ, 345: e7765 (2012).

New combinations of therapeutic agents that provide a beneficial effect in the treatment of cancers are desirable in order to prolong patient's lives while maintaining a high quality of life. New combinations may provide an increased benefit as compared to each of the agents alone. In particular, combined treatment regimens may be helpful for patients suffering from disease conditions including proliferative disorders, autoimmune diseases, inflammatory diseases, fibrotic diseases and kidney diseases, and could potentially even decrease the rate of relapse or overcome the resistance to a particular anticancer agent sometimes seen in these patients. This is especially true in the case where the cancers may be resistant or refractory to currently available therapeutic regimens.

Thus, there is a need for new cancer treatment regimens, including combination therapies.

### SUMMARY

The invention is set out in the appended set of claims. Any embodiment not falling under the scope of the appended claims does not form part of the invention.

Any references in the description to methods of treatment by therapy refer to the compounds, pharmaceutical compositions and medicaments of the present invention for use in those methods.

In one aspect, the present disclosure relates to methods of treating cancer comprising administering a STING agonist, a checkpoint inhibitor, and radiation in combination to a subject in need of such treatment.

In one aspect, the present disclosure relates to methods of treating a patient having cancer, comprising administering to a patient in need of said treating a combination of Compound No. 14, having the following structure: 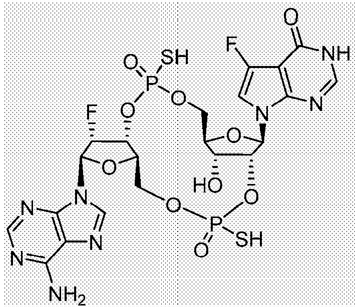 or a pharmaceutically acceptable salt thereof, a checkpoint inhibitor, and radiation.

In another aspect, the present disclosure relates to methods of treating a patient having cancer, comprising administering to a patient that has undergone radiation therapy Compound No. 14 and a checkpoint inhibitor. In some embodiments, Compound No. 14 and the checkpoint inhibitor are administered to a patient from 1 day to 3 months after the patient received treatment with radiation.

The following embodiments refer to both aspects of the invention.

In some embodiments, the checkpoint inhibitor is selected from the group consisting of anti-PD-1 antibodies.

In some embodiments, the anti-PD-1 antibody is selected from the group consisting of nivolumab, pembrolizumab, lambrolizumab, pidilizumab, BMS-936559, and AMP-224.

In some embodiments, the radiation is particle radiation.

In some embodiments, the radiation is administered by external beam radiation.

In some embodiments, Compound No. 14 or a pharmaceutically acceptable salt thereof, is administered orally.

In some embodiments, Compound No. 14 or a pharmaceutically acceptable salt thereof, is administered intravenously.

In some embodiments, Compound No. 14 or a pharmaceutically acceptable salt thereof, is administered by intravenous infusion.

In some embodiments, the checkpoint inhibitor is administered intravenously.

In some embodiments, the checkpoint inhibitor is administered by intravenous infusion.

In some embodiments, the checkpoint inhibitor is administered by subcutaneous injection.

In some embodiments, the checkpoint inhibitor is administered subcutaneously.

In some embodiments, Compound No. 14 and the checkpoint inhibitor are administered concurrently.

In some embodiments, Compound No. 14 and the checkpoint inhibitor are administered sequentially in separate pharmaceutical compositions.

In some embodiments, the radiation, Compound No. 14, and checkpoint inhibitor are administered sequentially.

In some embodiments, the radiation is administered before Compound No. 14 and the checkpoint inhibitor.

In some embodiments, the cancer is a PD-1 positive cancer, a PD-L1 positive cancer, or a CTLA-4 positive cancer.

In some embodiments, the cancer is a solid tumor or a hematological malignancy. In some embodiments, the cancer is a metastatic solid tumor. In some embodiments, the cancer is an advanced solid tumor.

In some embodiments, the cancer is melanoma, lung cancer, renal cancer, lymphoma, head and neck cancer, urothelial cancer, prostate cancer, bladder cancer, breast cancer, gastric cancer, colorectal cancer, leukemia, cervical cancer, microsatellite instability-high cancer, hepatocellular carcinoma, or Merkel cell carcinoma.

In some embodiments, the melanoma is metastatic melanoma, unresectable melanoma, or cutaneous melanoma.

In some embodiments, the lung cancer is non-small cell lung cancer or small cell lung cancer.

In some embodiments, the non-small cell lung cancer is metastatic non-small cell lung cancer, metastatic squamous non-small cell lung cancer, or metastatic nonsquamous non-small cell lung cancer.

In some embodiments, the renal cancer is renal cell carcinoma.

In some embodiments, the lymphoma is classical Hodgkin lymphoma or primary mediastinal large B-cell lymphoma.

In some embodiments, the head and neck cancer is head and neck squamous cell carcinoma.

In some embodiments, the urothelial cancer is urothelial carcinoma.

In some embodiments, the prostate cancer is hormone-refractory prostate cancer.

In some embodiments, the gastric cancer is gastroesophageal junction adenocarcinoma.

In some embodiments, the cancer is microsatellite instability-high cancer.

In some embodiments, the cancer is triple negative breast cancer.

In some embodiments, the cancer is a metastatic solid tumor.

In some embodiments, the checkpoint inhibitor is administered once every twelve weeks, once every four weeks, once every three weeks, once every two weeks, once every week, twice a week, three times a week, or daily.

In some embodiments, the checkpoint inhibitor is administered once every two weeks.

In some embodiments, the checkpoint inhibitor is administered once every three weeks.

In some embodiments, the checkpoint inhibitor is administered once every four weeks.

In some embodiments, the checkpoint inhibitor is administered once every twelve weeks.

In some embodiments, the checkpoint inhibitor is administered on Day 1 or Day 2 of a treatment cycle.

In some embodiments, the checkpoint inhibitor is administered on Day 1 of a treatment cycle.

In some embodiments, Compound No. 14 or a pharmaceutically acceptable salt thereof, is administered once every two weeks, once every week, twice a week, three times a week, or daily.

In some embodiments, Compound No. 14 or a pharmaceutically acceptable salt thereof, is administered twice a week.

In some embodiments, Compound No. 14 or a pharmaceutically acceptable salt thereof, is administered once every week.

In some embodiments, Compound No. 14 or a pharmaceutically acceptable salt thereof, is administered on days 1, 8, and 15 of a treatment cycle.

In some embodiments, Compound No. 14 or a pharmaceutically acceptable salt thereof, is administered on days 1, 4, 8, and 11 of a treatment cycle.

In some embodiments, Compound No. 14 or a pharmaceutically acceptable salt thereof, is administered on days 1 and 8 of a treatment cycle.

The following embodiments relate to the aspect of the invention where radiation is administered.

In some embodiments, the radiation is administered between Day -8 and Day -1 of a treatment cycle.

In some embodiments, the radiation is administered between Day -7 and Day -1 of a treatment cycle.

In some embodiments, the radiation is administered on Day 1 of a treatment cycle.

In some embodiments, the treatment cycle is 14 days, 21 days, 28 days, or 84 days.

In some embodiments, the treatment cycle is 21 days.

In some embodiments, Compound No. 14 is administered on Days 1, 8, and 15 of a treatment cycle and the checkpoint inhibitor is administered on Day 1 of a treatment cycle.

In some embodiments, Compound No. 14 is administered on Days 1, 8, and 15 of a treatment cycle, the checkpoint inhibitor is administered on Day 1 of a treatment cycle, and the radiation is administered between Day -8 and Day -1 of a treatment cycle.

In some embodiments, Compound No. 14 is administered on Days 1, 8, and 15 of a treatment cycle, the checkpoint inhibitor is administered on Day 1 of a treatment cycle, and the radiation is administered between Day -7 and Day -1 of a treatment cycle.

In some embodiments, Compound No. 14 is administered on Days 1, 8, and 15 of a treatment cycle, the checkpoint inhibitor is administered on Day 1 of a treatment cycle, and the radiation is administered at least 40 hours before administration of the checkpoint inhibitor or Compound No. 14.

In some embodiments, the radiation is administered at least 40 hours before administration of the checkpoint inhibitor or Compound No. 14.

In some embodiments, the radiation is administered at a fraction dose of about 5 Gy to about 20 Gy, or about 6 Gy to about 18 Gy, or about 8 Gy to about 16 Gy.

In some embodiments, the radiation is administered at a fraction dose of about 5 Gy to about 10 Gy, or about 15 Gy to about 20 Gy.

In some embodiments, the radiation is administered at a fraction dose of about 8 Gy or about 16 Gy.

In some embodiments, the radiation is administered in 1 fraction, or in 2 fractions, or in 3 fractions, or in 4 fractions, or in 5 fractions.

In some embodiments, the radiation is administered in 1 fraction or in 3 fractions.

In some embodiments, the radiation is administered at a fraction dose of about 8 Gy for 1 fraction, or about 8 Gy for 3 fractions, or about 16 Gy for 1 fraction.

In some embodiments, the checkpoint inhibitor is administered in an amount of 200 mg; Compound No. 14 is administered in an amount of from 0.05 mg to 2.5 mg, or in an amount of from 0.2 mg to 1.2 mg; and the radiation is administered at a fraction dose of about 8 Gy for 1 fraction, or about 8 Gy for 3 fractions, or about 16 Gy for 1 fraction.

In some embodiments, Compound No. 14, or a pharmaceutically acceptable salt thereof, is administered once every two weeks, once every week, twice a week, three times a week, daily, on days 1, 4, 8, and 11 of a 21 day cycle, or on days 1, 8, and 15 of a 21 day cycle; the checkpoint inhibitor is separately administered once every twelve weeks, once every four weeks, once every three weeks, once every two weeks, once every week, twice a week, three times a week, or daily; and the radiation is administered between day -8 and day -1 of a 21 day cycle, between day -7 and day -1 of a 21 day cycle, on day 1 of a 21 day cycle, or at least 40 hours before administration of the checkpoint inhibitor or Compound No. 14.

The following embodiments relate to both aspects of the invention.

In some embodiments, the checkpoint inhibitor is administered in an amount of 200 mg.

In some embodiments, Compound No. 14 is administered in an amount of 0.05 mg, 0.1 mg, 0.2 mg, 0.4 mg, 0.8 mg, 1.2 mg, 1.6 mg, 2.0 mg, or 2.5 mg.

In some embodiments, Compound No. 14 is administered in an amount of from 0.05 mg to 3.5 mg, or in an amount of from 0.1 mg to 3.5 mg, or in an amount of from 0.2 mg to 2.5 mg, or in an amount of from 0.05 mg to 1.2 mg, or in an amount of from 0.1 mg to 1.2 mg, or in an amount of from 0.2 mg to 1.2 mg.

In some embodiments, Compound No. 14, or a pharmaceutically acceptable salt thereof, and the checkpoint inhibitor are administered simultaneously once every twelve weeks, once every four weeks, once every three weeks, once every two weeks, once every week, twice a week, three times a week, daily, on days 1, 4, 8, and 11 of a 21 day cycle, or on days 1, 8, and 15 of a 21 day cycle.

In some embodiments, Compound No. 14, or a pharmaceutically acceptable salt thereof, is administered once every two weeks, once every week, twice a week, three times a week, daily, on days 1, 4, 8, and 11 of a 21 day cycle, or on days 1, 8, and 15 of a 21 day cycle; and the checkpoint inhibitor is separately administered once every twelve weeks, once every four weeks, once every three weeks, once every two weeks, once every week, twice a week, three times a week, or daily.

In one aspect, the present disclosure relates to a kit comprising a medicament for use in treating cancer in a subject in need of such treatment. The kit comprises a medicament comprising a STING agonist, and instructions for administering the STING agonist, the one or more checkpoint inhibitors, and radiation; or the kit comprises a medicament comprising the one or more checkpoint inhibitors, and instructions for administering the one or more checkpoint inhibitors, a STING agonist, and radiation. The kit can contain both a medicament comprising a STING agonist and a medicament comprising one or more checkpoint inhibitors, and instructions for administering the STING agonist, the one or more checkpoint inhibitors, and radiation. The kit can also comprise one or more additional therapeutic agents.

In one aspect, the present disclosure relates to a medicament for use in treating cancer in a subject in need of such treatment. The medicament comprises a STING agonist and one or more checkpoint inhibitors. The medicament can also comprise one or more additional therapeutic agents.

### BRIEF DESCRIPTION OF THE FIGURES

FIG. 1 shows tumor growth curves as a function of time for vehicle, Compound No. 14, Radiation only, and combination treatment groups in EMT6 syngeneic tumor models from study 1. Compound No. 14 was administered at 1.0 mg/kg three times on every third day (Q3D x 3). Radiation was administered at 10 Gy as a single dose.
FIG. 2 shows tumor growth curves as a function of time for vehicle, Compound No. 14, Radiation only, and combination treatment groups in EMT6 syngeneic tumor models from study 2. Compound No. 14 was administered at 1.0 mg/kg three times on every third day (Q3D x 3). Radiation was administered at 10 Gy as a single dose.

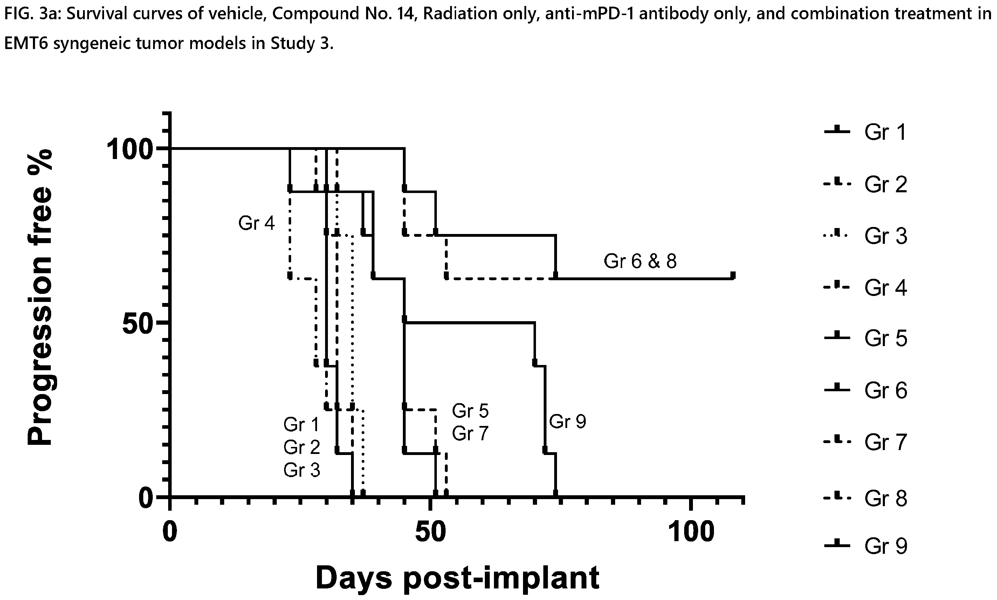

FIG. 3a shows survival curves as a function of time for vehicle, Compound No. 14, Radiation only, anti-mPD-1 antibody only, and combination treatment in EMT6 syngeneic tumor models from study 3. Compound No. 14 was administered at 0.25 mg/kg or 1.0 mg/kg three times on every third day (Q3D x 3). The anti-mPD-1 antibody or isotype control was administered at 10 mg/kg three times on every third day (Q3D x 3). Radiation was administered at 8 Gy for 3 doses. Group 1 represents administration of vehicle (PBS), isotype control, and mock radiation. Group 2 represents administration of Compound No. 14 (1 mg/kg), isotype control, and mock radiation. Group 3 represents administration of Compound No. 14 (0.25 mg/kg), isotype control, and mock radiation. Group 4 represents administration of vehicle, anti-mPD-1 antibody, and mock radiation. Group 5 represents administration of vehicle, isotype control, and radiation (8 Gy x 3). Group 6 represents administration of Compound No. 14 (1 mg/kg), isotype control, and radiation (8 Gy x 3). Group 7 represents administration of vehicle, anti-mPD-1 antibody, and radiation (8 Gy x 3). Group 8 represents administration of Compound No. 14 (1 mg/kg), anti-mPD-1 antibody, and radiation (8 Gy x 3). Group 9 represents administration of Compound No. 14 (0.25 mg/kg), anti-mPD-1 antibody, and radiation (8 Gy x 3).
FIG. 3b shows tumor growth curves as a function of time for vehicle, Compound No. 14, Radiation only, anti-mPD-1 antibody only, isotype control only, and combination treatment groups in EMT6 syngeneic tumor models from study 3. Vehicle (PBS) or Compound No. 14 was administered at 0.25 mg/kg or 1.0 mg/kg three times on every third day (Q3D x 3). The anti-mPD-1 antibody or isotype control was administered at 10 mg/kg three times on every third day (Q3D x 3). Radiation was administered at 8 Gy for 3 doses.
FIG. 4a shows survival curves as a function of time for vehicle, Compound No. 14, anti-mPD-1 antibody only, Radiation only, and combination treatments in CT26 tumor models from study 4.
FIG. 4b shows tumor growth curves as a function of time for vehicle, Compound No. 14, anti-mPD-1 antibody only, Radiation only, and combination treatment groups in CT26 tumor models from study 4.
FIG. 5a shows survival curves as a function of time for vehicle, Compound No. 14, Radiation only, and combination treatment in B16F10 ova tumor models from study 5.
FIG. 5b shows tumor growth curves as a function of time for vehicle, Compound No. 14, Radiation only, and combination treatment groups B16F10 ova tumor models from study 5.

### DETAILED DESCRIPTION

### Definitions and Abbreviations

To facilitate an understanding of the present disclosure, a number of abbreviations, terms, and phrases are defined below.
- AUC: area under the plasma concentration versus time curve
- BSA: body surface area
- CR: complete response
- MTD: maximum tolerated dose
- STING: stimulator of interferon genes
- PR: partial response
- BIW: twice weekly
- QW: once weekly
- Q2W: once every 2 weeks
- Q3D: every third day
- QD: once daily
- Q: Every
- NSCLC: non-small cell lung cancer
- SCLC: small cell lung cancer
- CPI: checkpoint inhibitor

Unless defined otherwise, all technical and scientific terms used herein have the same meaning as is commonly understood by one of skill in the art to which this disclosure belongs.

As used herein, the term "cancer" refers to a cellular disorder characterized by uncontrolled or dysregulated cell proliferation, decreased cellular differentiation, inappropriate ability to invade surrounding tissue, and/or ability to establish new growth at ectopic sites. The term "cancer" includes solid tumors and non-solid tumors, such as, for example, hematological tumors. The term "cancer" encompasses diseases of skin, tissues, organs, bone, cartilage, blood, and vessels. The term "cancer" further encompasses primary and metastatic cancers.

The term "radiation" and the term "radiotherapy" are used interchangeably throughout the disclosure.

The term "PD-1" (also known as programmed cell death protein 1, PDCD1, CD279, SLEB2, or SLE1) refers to any native PD-1, unless otherwise indicated. The term "PD-1" encompasses "full-length," unprocessed PD-1 as well as any form of PD-1 that results from processing within the cell. The term also encompasses naturally occurring variants of PD-1, e.g., splice variants, allelic variants, and isoforms.

The term "PD-L1" (also known as programmed cell death 1 ligand) refers to any native PD-L1, unless otherwise indicated. The term "PD-L1" encompasses "full-length," unprocessed PD-L1 as well as any form of PD-L1 that results from processing within the cell. The term also encompasses naturally occurring variants of PD-L1, e.g., splice variants, allelic variants, and isoforms.

The term "CTLA-4" (also known as cytotoxic T-lymphocyte-associated antigen 4) refers to any native CTLA-4, unless otherwise indicated. The term " CTLA-4" encompasses "full-length," unprocessed CTLA-4 as well as any form of CTLA-4 that results from processing within the cell. The term also encompasses naturally occurring variants of CTLA-4, e.g., splice variants, allelic variants, and isoforms.

The term "antibody" means an immunoglobulin molecule that recognizes and specifically binds to a target, such as a protein, polypeptide, peptide, carbohydrate, polynucleotide, lipid, or combinations of the foregoing through at least one antigen recognition site within the variable region of the immunoglobulin molecule. As used herein, the term "antibody" encompasses intact polyclonal antibodies, intact monoclonal antibodies, antibody fragments (such as Fab, Fab', F(ab')2, and Fv fragments), single chain Fv (scFv) mutants, multispecific antibodies such as bispecific antibodies generated from at least two intact antibodies, chimeric antibodies, humanized antibodies, human antibodies, fusion proteins comprising an antigen determination portion of an antibody, and any other modified immunoglobulin molecule comprising an antigen recognition site so long as the antibodies exhibit the desired biological activity. An antibody can be of any of the five major classes of immunoglobulins: IgA, IgD, IgE, IgG, and IgM, or subclasses (isotypes) thereof (e.g., IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well known subunit structures and three-dimensional configurations. Antibodies can be naked or conjugated to other molecules such as toxins, radioisotopes, etc.

A "blocking" antibody or an "antagonist" antibody is one which inhibits or reduces biological activity of the antigen it binds, such as, e.g., PD-1, PD-L1, or CTLA-4. In a certain embodiment, blocking antibodies or antagonist antibodies substantially or completely inhibit the biological activity of the antigen. Desirably, the biological activity is reduced by 10%, 20%, 30%, 50%, 70%, 80%, 90%, 95%, or even 100%.

The term "anti-PD-1 antibody" or "an antibody that binds to PD-1" refers to an antibody that is capable of binding PD-1 with sufficient affinity such that the antibody is useful as a diagnostic and/or therapeutic agent in targeting PD-1. The extent of binding of an anti-PD-1 antibody to an unrelated, non-PD-1 protein is less than about 10% of the binding of the antibody to PD-1 as measured, e.g., by a radioimmunoassay (RIA). In certain embodiments, an antibody that binds to PD-1 has a dissociation constant (Kd) of ≤1 µM, ≤100 nM, ≤10 nM, ≤1 nM, or ≤0.1 nM.

The term "anti-PD-L1 antibody" or "an antibody that binds to PD-L1" refers to an antibody that is capable of binding PD-L1 with sufficient affinity such that the antibody is useful as a diagnostic and/or therapeutic agent in targeting PD-L1. The extent of binding of an anti-PD-L1 antibody to an unrelated, non-PD-L1 protein is less than about 10% of the binding of the antibody to PD-L1 as measured, e.g., by a radioimmunoassay (RIA). In certain embodiments, an antibody that binds to PD-L1 has a dissociation constant (Kd) of ≤1 µM, ≤100 nM, ≤10 nM, ≤1 nM, or ≤0.1 nM.

The term "anti-CTLA-4 antibody" or "an antibody that binds to CTLA-4" refers to an antibody that is capable of binding CTLA-4 with sufficient affinity such that the antibody is useful as a diagnostic and/or therapeutic agent in targeting CTLA-4. The extent of binding of an anti-CTLA-4 antibody to an unrelated, non-CTLA-4 protein is less than about 10% of the binding of the antibody to CTLA-4 as measured, e.g., by a radioimmunoassay (RIA). In certain embodiments, an antibody that binds to CTLA-4 has a dissociation constant (Kd) of ≤1 µM, ≤100 nM, ≤10 nM, ≤1 nM, or ≤0.1 nM.

A "monoclonal antibody" refers to a homogeneous antibody population involved in the highly specific recognition and binding of a single antigenic determinant, or epitope. This is in contrast to polyclonal antibodies that typically include different antibodies directed against different antigenic determinants. The term "monoclonal antibody" encompasses both intact and full-length monoclonal antibodies as well as antibody fragments (such as Fab, Fab', F(ab')2, Fv), single chain (scFv) mutants, fusion proteins comprising an antibody portion, and any other modified immunoglobulin molecule comprising an antigen recognition site. Furthermore, "monoclonal antibody" refers to such antibodies made in any number of manners including but not limited to by hybridoma, phage selection, recombinant expression, and transgenic animals.

The term "chimeric antibodies" refers to antibodies wherein the amino acid sequence of the immunoglobulin molecule is derived from two or more species. Typically, the variable region of both light and heavy chains corresponds to the variable region of antibodies derived from one species of mammals (e.g., mouse, rat, rabbit, etc.) with the desired specificity, affinity, and capability while the constant regions are homologous to the sequences in antibodies derived from another (usually human) to avoid eliciting an immune response in that species.

As used herein, the term "effective amount" or "therapeutically effective amount" refers to an amount of a compound, or combination of one or more compounds that, when administered (either sequentially or simultaneously) elicits the desired biological or medicinal response, e.g., either destroys the target cancer cells or slows or arrests the progression of the cancer in a patient. The therapeutically effective amount may vary depending upon the intended application (in vitro or in vivo), or the patient and disease condition being treated, e.g., the weight and age of the patient, the severity of the disease condition, the manner of administration and the like, which may readily be determined by one skilled in the art. The term also applies to a dose that will induce a particular response in target cells, e.g., reduction of platelet adhesion and/or cell migration. For example, in some embodiments, the "therapeutically effective amount" as used herein refers to the amount of Compound No. 14 or a pharmaceutically acceptable salt thereof, and the amount of checkpoint inhibitor that, when administered separately or in combination, have a beneficial effect. In some embodiments, the combined effect is additive. In some embodiments, the combined effect is synergistic. Further, it will be recognized by one skilled in the art that in the case of combination therapy, the amount of Compound No. 14 or a pharmaceutically acceptable salt thereof and/or the amount of the checkpoint inhibitor may be used in a "sub-therapeutic amount", i.e., less than the therapeutically effective amount of Compound No. 14 or a pharmaceutically acceptable salt thereof, or the checkpoint inhibitor alone.

In any form or composition, the administered dose(s) or the therapeutically effective (total) amount may be expressed as amount(s) of therapeutic substance(s) per patient as either based on (i) BSA, e.g., as mg/m², or (ii) amount, e.g., as mg.

The term "about" refers to approximately, in the region of, roughly, or around. When the term "about" is used in conjunction with a number or a numerical range, it means that the number or numerical range referred to is an approximation within experimental variability (or within statistical experimental error), and thus the number or numerical range may vary from, for example, between 1% and 15% of the stated number or numerical range. In general, the term "about" is used herein to modify a numerical value above and below the stated value by a variance of ± 10%.

As used herein, "patient" generally means a mammal (e.g., human) who has been diagnosed with, exhibits symptoms of, or is otherwise believed to be afflicted with a disease, disorder, or condition (such as cancer).

As used herein, "body surface area" (BSA) is calculated using a standard nomogram, e.g., $BSA \left({m}^{2}\right) = \sqrt{\frac{Ht \left(cm\right) \times Wt \left(kg\right)}{3600}} or BSA = \sqrt{\frac{Ht \left(in\right) \times Wt \left(lb\right)}{3131}} .$

The term "combination administration," "administered in combination," and "administering a combination" refers to administering of more than one pharmaceutically active ingredients (including, but not limited to, Compound No. 14 or a pharmaceutically acceptable salt thereof, a checkpoint inhibitor, or radiation as disclosed herein) to a patient. Combination administration may refer to simultaneous administration or may refer to sequential administration of Compound No. 14 or a pharmaceutically acceptable salt thereof, a checkpoint inhibitor, and radiation as disclosed herein.

The terms "simultaneous" and "simultaneously" refer to the administration of Compound No. 14 or a pharmaceutically acceptable salt thereof, and a checkpoint inhibitor as disclosed herein, or the administration of Compound No. 14 or a pharmaceutically acceptable salt thereof, a checkpoint inhibitor, and radiation, or any combination thereof, as disclosed herein, to a patient at the same time, or at two or three different time points that are separated by no more than 2 hours. The simultaneous administration of Compound No. 14 or a pharmaceutically acceptable salt thereof, and a checkpoint inhibitor may be in a single dosage form or in separate dosage forms.

The terms "sequential" and "sequentially" refer to the administration of Compound No. 14 or a pharmaceutically acceptable salt thereof, and a checkpoint inhibitor as disclosed herein, or to the administration of Compound No. 14 or a pharmaceutically acceptable salt thereof, a checkpoint inhibitor, and radiation, or any combination thereof, as disclosed herein, to a patient at two or three different time points that are separated by more than 2 hours, e.g., about 3 hours, about 4 hours, about 5 hours, about 8 hours, about 12 hours, 1 day, 2 days, 3 days, 4 days, 5 days, 6 days, 7 days or even longer.

The term "intermission" refers to a period that is subsequent to the administration of one or more particular pharmaceutically active ingredients to a patient in an intermittent regimen. Intermission refers to a rest period wherein a particular pharmaceutically active ingredient is not administered for at least one day.

The term "synergistic effect" refers to a situation where the combination of two or more agents produces a greater effect than the sum of the effects of each of the individual agents. The term encompasses not only a reduction in symptoms of the disorder to be treated, but also an improved side effect profile, improved tolerability, improved patient compliance, improved efficacy, or any other improved clinical outcome.

The term "fraction dose" refers to the dose of radiation administered in each individual fraction.

As used herein, the illustrative terms "include", "such as", "for example" and the like (and variations thereof, e.g., "includes" and "including", "examples"), unless otherwise specified, are intended to be non-limiting. That is, unless explicitly stated otherwise, such terms are intended to imply "but not limited to", *e.g.,* "including" means including but not limited to.

Unless otherwise stated, structures depicted herein are meant to include chemical entities which differ only in the presence of one or more isotopically enriched atoms. For example, chemical entities having the present structure except for the replacement of a hydrogen atom by a deuterium or tritium, or the replacement of a carbon atom by a ¹³C- or ¹⁴C-enriched carbon are within the scope of the invention.

Unless stereochemical configuration is denoted, structures depicted herein are meant to include all stereochemical forms of the structure, i.e., the R and S configurations for each asymmetric center. Therefore, unless otherwise indicated, single stereochemical isomers as well as enantiomeric, racemic and diastereomeric mixtures of the present chemical entities are within the scope of the invention. When a stereochemical configuration is denoted for a compound, the diastereoisomeric or enantiomeric excess of the compound is at least 99.0%, 99.5%, 99.6%, 99.7%, 99.8% or 99.9%.

### STING Agonist

The present disclosure provides a combination treatment for patients with cancer . The combination treatment includes, *inter alia,* administering to a subject in need thereof a therapeutically effective amount of at least one STING agonist.

In some embodiments, the STING agonist is a compound of Formula I, or a pharmaceutically acceptable salt thereof, having the following structure: 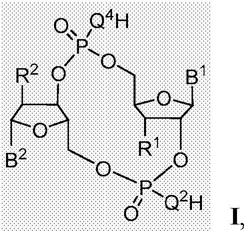 wherein R¹ and R² are each independently a hydroxy group or a halogen atom;
B¹ is: 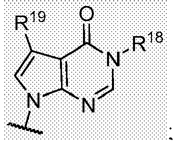
R¹⁸ is hydrogen or C₁₋₆ alkyl;
R¹⁹ is a halogen atom;
B² is: 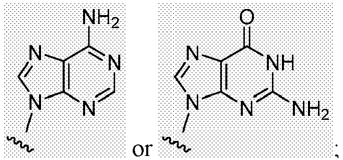 and
Q² and Q⁴ are each independently an oxygen atom or a sulfur atom.

In some embodiments, the STING agonist is Compound No. 14, or a pharmaceutically acceptable salt thereof, having the following structure:

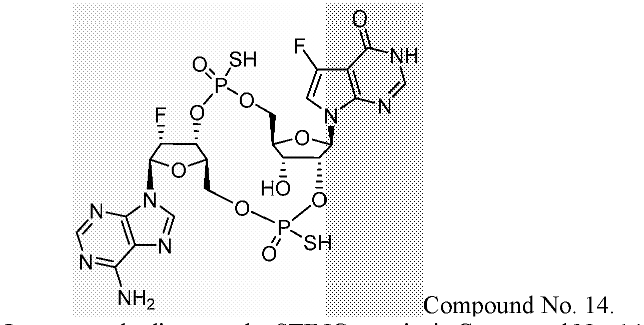

In some embodiments, the STING agonist is Compound No. 14, or a pharmaceutically acceptable salt thereof.

In some embodiments, the STING agonist is Compound No. 14.

Particular STING agonists, as disclosed herein, are described, for example, in PCT Application Publication No. WO 2018/100558. They may be prepared by methods known to one skilled in the art and/or according to the methods described in WO 2018/100558.

In some embodiments, the STING agonist is Compound No. 14 or a crystalline form thereof.

### Checkpoint Inhibitors

The present disclosure provides a combination treatment that includes, *inter alia,* administering to a subject in need thereof a therapeutically effective amount of at least one checkpoint inhibitor (e.g., nivolumab, pembrolizumab, atezolizumab, durvalumab, avelumab, and ipilimumab). In some embodiments, the checkpoint inhibitor is an anti-PD-1 antibody.

PD-1 is a type I transmembrane protein that is one of the major immune checkpoint molecules (Blank et al., 2005, Cancer Immunotherapy, 54:307-314). PD-1 is primarily expressed on activated T cells, and it interacts with the ligands PD-L1 (B7-Hl or CD274) and PD-L2 (B7-DC or CD273) to induce an inhibitory signal resulting in reduced T cell proliferation, cytokine production, and cytotoxic activity (Freeman et al., 2000, J. Exp. Med., 192:1027-34).

In some embodiments, the anti-PD-1 antibody is a fully human monoclonal antibody. In some embodiments, the anti-PD-1 antibody is a humanized IgG monoclonal antibody.

In some embodiments, the anti-PD-1 antibody is a full length (intact) antibody. In some embodiments, the anti-PD-1 antibody consists of anti-PD-1 binding fragments, including, but not limited to, Fab, Fab', F(ab')₂, and Fv fragments, single chain Fv fragments, and single chain domain fragments.

In some embodiments, the anti-PD-1 antibody is a derivatized antibody. In some embodiments, the anti-PD-1 antibody is derivatized by glycosylation, acetylation, pegylation, phosphorylation, and amidation. In some embodiments, the anti-PD-1 antibody is derivatized by known protecting/blocking groups, proteolytic cleavage, linkage to a cellular ligand or other protein. In some embodiments, the derivatized anti-PD-1 antibody can contain one or more non-natural amino acids, e.g., using ambrx technology (See, e.g., Wolfson, 2006, Chem. Biol. 13(10):1011-2).

In some embodiments, the anti-PD-1 antibody is nivolumab.

Nivolumab is a human monoclonal antibody that blocks the interaction between PD-1 and its ligands, PD-L1 and PD-L2. Nivolumab is an IgG4 kappa immunoglobulin that has a calculated molecular mass of 146 kDa. It is expressed in a recombinant Chinese Hamster Ovary (CHO) cell line. Nivolumab is approved by the FDA for treating unresectable or metastatic melanoma, melanoma, metastatic non-small cell lung cancer, advanced renal cell carcinoma, classical Hodgkin lymphoma, squamous cell carcinoma of the head and neck, urothelial carcinoma, microsatellite instability-high (MSI-H) or mismatch repair deficient (dMMR) metastatic colorectal cancer, and hepatocellular carcinoma. Nivolumab is commercially available as Opdivo^{®}.

In some embodiments, the anti-PD-1 antibody is pembrolizumab.

Pembrolizumab is a humanized monoclonal antibody that blocks the interaction between PD-1 and its ligands, PD-L1 and PD-L2. Pembrolizumab is an IgG4 kappa immunoglobulin with an approximate molecular mass of 149 kDa. Pembrolizumab is produced in recombinant Chinese hamster ovary (CHO) cells. Pembrolizumab is approved by the FDA for treating melanoma, non-small cell lung cancer, head and neck cancer, classical Hodgkin lymphoma, primary mediastinal large B-cell lymphoma, urothelial carcinoma, microsatellite instability-high cancer, gastric cancer, and cervical cancer. Pembrolizumab is commercially available as Keytruda^{®}.

In some embodiments, the anti-PD-1 antibody is cemiplimab.

Cemiplimab is a human monoclonal antibody that binds to PD-1 and blocks its interaction with PD-L1 and PD-L2. Cemiplimab is an IgG4 immunoglobulin with an approximate molecular mass of 146 kDa. Cemiplimab is produced by recombinant DNA technology in Chinese hamster ovary (CHO) cell suspension. Cemiplimab is approved by the FDA for treating metastatic cutaneous squamous cell carcinoma (CSCC) or locally advanced CSCC who are not candidates for curative surgery or curative radiation. Cemiplimab is commercially available as Libtayo^{®}.

Additional anti-PD-1 antibodies include, for example, pidilizumab (Medivation), BMS-936559 (Bristol-Myers Squibb), and AMP-224.

In some embodiments, the anti-PD-1 antibody used in the methods (and kits) described herein is nivolumab or an anti-PD-1 antibody that binds to the same epitope as nivolumab. In some embodiments, the anti-PD-1 antibody is nivolumab.

In some embodiments, the anti-PD-1 antibody used in the methods (and kits) described herein is pembrolizumab or an anti-PD-1 antibody that binds to the same epitope as pembrolizumab. In some embodiments, the anti-PD-1 antibody is pembrolizumab.

### Radiation

In some embodiments, the radiation is photon radiation (x-rays and gamma rays). In such embodiments, the photons are generated as a high energy photon beam from radioactive sources such as cobalt or a linear accelerator.

In some embodiments, the radiation is particle radiation (such as electrons, protons, neutrons, carbon ions, alpha particles, and beta particles). Particle radiation can be produced by linear accelerators. In some embodiments, the radiation is an electron beam. In some embodiments, the radiation is a proton beam. In some embodiments, the radiation is a neutron beam.

In some embodiments, the radiation is delivered by external beam radiation. In some embodiments, the external beam radiation is three-dimensional conformal radiation therapy (3D-CRT). In some embodiments, the external beam radiation is intensity modulated radiation therapy (IMRT). In some embodiments, the external beam radiation is image-guided radiation therapy (IGRT). In some embodiments, the external beam radiation is intensity modulated proton therapy (IMPT). In some embodiments, the external beam radiation is stereotactic radiosurgery (SRS). In some embodiments, the external beam therapy is fractionated stereotactic radiotherapy. In some embodiments, the external beam radiation is stereotactic body radiation therapy (SBRT). Examples of machines that deliver SBRT are Gamma Knife^{®}, X-Knife^{®}, CyberKnife^{®}, and Clinac^{®}. In some embodiments, the radiation can be administered using a three dimensional conformal or stereotactic body radiation therapy delivery.

In some embodiments the radiation is delivered by internal radiation therapy (brachytherapy). In such embodiments, the internal radiation therapy is interstitial radiation, for example, using small pellets, seeds, wires or tubes placed close to the cancer or tumor site. In such embodiments, the internal radiation therapy is intracavitary radiation, for example using a container of radioactive material that is placed in a body cavity.

### Methods of Treating Cancer

In some embodiments, the present disclosure relates to a method of treating cancer in a patient by administering to a patient in need of said treating a combination of a STING agonist or pharmaceutically acceptable salt thereof, one or more checkpoint inhibitors, and radiation.

In some embodiments, the present disclosure relates to a method of treating cancer by administering to a patient in need of said treating a combination of a STING agonist, a checkpoint inhibitor, and radiation.

In some embodiments, the present disclosure relates to a composition comprising a STING agonist for use in treating cancer in a patient, wherein the patient is also treated with a checkpoint inhibitor and radiation. In some aspects, the disclosure relates to a composition comprising a STING agonist for use in treating cancer in a patient, wherein the STING agonist is in combination with the checkpoint inhibitor and radiation. In some embodiments, the STING agonist can be administered simultaneously or sequentially with the checkpoint inhibitor, radiation, and combinations thereof.

In some embodiments, the present disclosure relates to methods of treating cancer comprising administering to a patient in need of such treatment, a therapeutically effective amount of a combination of a STING agonist, a checkpoint inhibitor, and radiation.

In some embodiments, the present disclosure relates to a method of treating cancer by administering to a patient a combination of Compound No. 14, or pharmaceutically acceptable salt thereof, a checkpoint inhibitor, and radiation.

In another aspect, the present disclosure relates to the use of Compound No. 14, or a pharmaceutically acceptable salt thereof, in combination with a checkpoint inhibitor and radiation for the treatment of cancer.

In some embodiments, the methods of treating cancer, as described herein, can include a combination of a STING agonist, a checkpoint inhibitor, radiation, and one or more additional therapeutic agents. In some embodiments, the one or more additional therapeutic agents can be chemotherapeutic agents. In some embodiments, the one or more additional therapeutic agents can include, but are not limited to, fludarabine, cyclophosphamide, doxorubicin, vincristine, methotrexate anthracycline-based chemotherapeutic agents, prednisone, methylprednisolone, glucocorticoids, Ibritumomab tiuxetan, acetaminophen, antihistamines, and combinations thereof. In another embodiment, the checkpoint inhibitor is coadministered with human hyaluronidase.

In some embodiments, the present disclosure relates to a method of treating a disorder, wherein the disorder is cancer.

In some embodiments, the cancer is a solid tumor. In some embodiments, the cancer is a metastatic solid tumor. In some embodiments, the cancer is an advanced solid tumor. Non-limiting examples of solid tumors include pancreatic cancer; bladder cancer, including invasive bladder cancer; colorectal cancer, including microsatellite instability-high (MSI-H) or mismatch repair deficient (dMMR) metastatic colorectal cancer; thyroid cancer; gastric cancer; breast cancer, including metastatic breast cancer and triple negative breast; prostate cancer, including androgen-dependent and androgen-independent prostate cancer; renal cancer, including, e.g., metastatic renal cell carcinoma and advanced renal cell carcinoma; urothelial carcinoma, including locally advanced or metastatic urothelial carcinoma; microsatellite instability-high cancer; liver cancer including e.g. hepatocellular carcinoma and intrahepatic bile duct cancer; lung and bronchus cancer including non-small cell lung cancer (NSCLC), squamous lung cancer, brochioloalveolar carcinoma (BAC), adenocarcinoma of the lung, and small cell lung cancer (SCLC); ovarian cancer including, e.g., progressive epithelial and primary peritoneal cancer; cervical cancer; uterine cancer including e.g. uterine corpus and uterine cervix; endometrial cancer; esophageal cancer; head and neck cancer, including, e.g., squamous cell carcinoma of the head and neck, nasopharyngeal caner, oral cavity and pharynx; melanoma, including unresectable or metastatic melanoma, and adjuvant treatment of melanoma; metastatic Merkel cell carcinoma; neuroendocrine cancer, including metastatic neuroendocrine tumors; brain cancer, including, e.g., glioma/glioblastoma, anaplastic oligodendroglioma, adult glioblastoma multiforme, and adult anaplastic astrocytoma; neuroendocrine cancer, including metastatic neuroendocrine tumors; bone cancer; gastro-esophageal junction cancer, and soft tissue sarcoma.

In some embodiments, the cancer is a hematological cancer. Non-limiting examples of hematologic malignancies include acute myeloid leukemia (AML); chronic myelogenous leukemia (CML), including accelerated CML and CML blast phase (CML-BP); acute lymphoblastic leukemia (ALL); chronic lymphocytic leukemia (CLL); Hodgkin's lymphoma (HL), including classical Hodgkin lymphoma; non-Hodgkin's lymphoma (NHL), including B-cell lymphoma, T-cell lymphoma, follicular lymphoma (FL), marginal zone lymphoma (MZL), mantle cell lymphoma (MCL), diffuse large B-cell lymphoma (DLBCL), primary mediastinal large B-cell lymphoma, and Burkitt lymphoma; multiple myeloma (MM); amyloidosis; Waldenstrom's macroglobulinemia; myelodysplastic syndromes (MDS), including refractory anemia (RA), refractory anemia with ringed siderblasts (RARS), (refractory anemia with excess blasts (RAEB), and RAEB in transformation (RAEB-T); and myeloproliferative syndromes. In some embodiments, the cancer is chronic lymphocytic leukemia (CLL), Hodgkin's lymphoma, or non-Hodgkin's lymphoma including follicular lymphoma (FL), marginal zone lymphoma (MZL), mantle cell lymphoma (MCL), Diffuse large B-cell lymphoma (DLBCL) and Burkitt lymphoma.

In some embodiments, the cancer is melanoma, lung cancer, renal cancer, lymphoma, head and neck cancer, urothelial cancer, prostate cancer, bladder cancer, breast cancer, gastric cancer, colorectal cancer, leukemia, cervical cancer, microsatellite instability-high cancer, hepatocellular carcinoma, or Merkel cell carcinoma.

In some embodiments, the melanoma is metastatic melanoma, unresectable melanoma, or cutaneous melanoma.

In some embodiments, the lung cancer is non-small cell lung cancer or small cell lung cancer.

In some embodiments, the non-small cell lung cancer is metastatic non-small cell lung cancer, metastatic squamous non-small cell lung cancer, or metastatic nonsquamous non-small cell lung cancer.

In some embodiments, the renal cancer is renal cell carcinoma.

In some embodiments, the lymphoma is classical Hodgkin lymphoma or primary mediastinal large B-cell lymphoma.

In some embodiments, the head and neck cancer is head and neck squamous cell carcinoma.

In some embodiments, the urothelial cancer is urothelial carcinoma.

In some embodiments, the prostate cancer is hormone-refractory prostate cancer.

In some embodiments, the gastric cancer is gastroesophageal junction adenocarcinoma.

In some embodiments, the cancer is microsatellite instability-high cancer.

In some embodiments, the cancer is triple negative breast cancer.

In some embodiments, the cancer is a metastatic solid tumor.

In some embodiments, the cancer is relapsed. In some embodiments, relapsed cancer is cancer which has returned after a period of time in which no cancer could be detected.

In some embodiments, the cancer is refractory. In some embodiments, refractory cancer does not respond to cancer treatment; it is also known as resistant cancer. In some embodiments, the cancer is resistant to rituximab. In some embodiments, the cancer does not respond to the treatment of rituximab. In some embodiments, the cancer is rituximab-resistant recurrent cancer. In some embodiments, the patient has become refractory to a rituximab-containing regimen. In some embodiments, the tumor is unresectable. In some embodiments, an unresectable tumor is unable to be removed by surgery. In some embodiments, the cancer has not been previously treated. In some embodiments, the cancer is locally advanced. In some embodiments, "locally advanced" refers to cancer that is somewhat extensive but still confined to one area. In some instances, "locally advanced" may refer to a small tumor that hasn't spread but has invaded nearby organs or tissues that make it difficult to remove with surgery alone. In some embodiments, the cancer is metastatic. In some embodiments, metastatic cancer is a cancer that has spread from the part of the body where it started (the primary site) to other parts of the body.

In some embodiments, the disorder is a STING-mediated disorder.

In some embodiments, the disorder is a PD-1-positive cancer. A PD-1-positive cancer includes a cancer where PD-1 is expressed on the cancer cells.

In some embodiments, the disorder is a PD-L1-positive cancer. A PD-L1-positive cancer includes a cancer where PD-L1 is expressed on the cancer cells.

In some embodiments, the disorder is a CTLA-4-positive cancer. A CTLA-4-positive cancer includes a cancer where CTLA-4 is expressed on the cancer cells.

### Medicament

In some embodiments, the present disclosure relates to a medicament for use in treating cancer in a patient in need of such treatment. In some embodiments, the medicament comprises a STING agonist, and is in single dosage form or in separate dosage forms. In some embodiments, the medicament comprises a STING agonist and a checkpoint inhibitor, and is in single dosage form or in separate dosage forms.

In some embodiments, the medicaments, as described herein, can include a combination of a STING agonist, a checkpoint inhibitor, and optionally one or more additional therapeutic agents.

In some embodiments, the present disclosure relates to the use of a STING agonist in the manufacture of a medicament for treating cancer, wherein the STING agonist is administered with a checkpoint inhibitor and radiation, and wherein the medicament is in single dosage form or in separate dosage forms. In some embodiments, the STING agonist is administered with a checkpoint inhibitor, radiation, and one or more additional therapeutic agents.

In some embodiments, the present disclosure relates to the use of a STING agonist for the manufacture of a medicament in treating cancer in a patient, wherein the patient is also treated with a checkpoint inhibitor, radiation, and optionally one or more additional therapeutic agents. In some embodiments, the STING agonist may be administered simultaneously or sequentially with the checkpoint inhibitor, radiation, or combinations thereof. In some aspects, the present disclosure relates to the use of a STING agonist for the manufacture of a medicament in treating cancer in a patient, wherein the STING agonist is in combination with a checkpoint inhibitor, radiation, and optionally one or more additional therapeutic agents. In some embodiments, the STING agonist is in the same composition as the checkpoint inhibitor. In some embodiments, the STING agonist is in a separate composition as the checkpoint inhibitor. In some embodiments, the STING agonist is in the same composition as one or more additional therapeutic agents. In some embodiments, the STING agonist is in the same composition as the checkpoint inhibitor, and optionally one or more additional therapeutic agents. In some embodiments, the STING agonist is in a separate composition as one or more additional therapeutic agents. In some embodiments, the STING agonist is in a separate composition as the checkpoint inhibitor, and optionally one or more additional therapeutic agents.

In another aspect, the present disclosure relates to the use of Compound No. 14, or a pharmaceutically acceptable salt thereof, in the manufacture of a medicament for treating cancer, wherein Compound No. 14 or a pharmaceutically acceptable salt thereof is administered with a checkpoint inhibitor, radiation, and optionally one or more additional therapeutic agents.

In some embodiments, the one or more additional therapeutic agents can be chemotherapeutic agents. In some embodiments, the one or more additional therapeutic agents can include, but are not limited to, fludarabine, cyclophosphamide, doxorubicin, vincristine, methotrexate anthracycline-based chemotherapeutic agents, prednisone, methylprednisolone, glucocorticoids, Ibritumomab tiuxetan, acetaminophen, antihistamines, and combinations thereof. In another embodiment, the checkpoint inhibitor is coadministered with human hyaluronidase.

### Administration of the Combination

Compound No. 14 or a pharmaceutically acceptable salt thereof, may be administered in combination with the checkpoint inhibitor, radiation, and optionally one or more additional therapeutic agents, in a single dosage form or as a separate dosage forms. In some embodiments, when administered as a separate dosage form, the checkpoint inhibitor may be administered prior to, at the same time as, or following administration of Compound No. 14 or a pharmaceutically acceptable salt thereof, and prior to, at the same time as, or following administration of radiation. In some embodiments, when administered as a separate dosage form, one or more doses of Compound No. 14 or a pharmaceutically acceptable salt thereof, may be administered prior to the checkpoint inhibitor and radiation. In some embodiments, the checkpoint inhibitor is administered prior to the administration of Compound No. 14 or a pharmaceutically acceptable salt thereof, and prior to the radiation. In some embodiments, the radiation is administered prior to the checkpoint inhibitor or prior to Compound No. 14 or a pharmaceutically acceptable salt thereof. In some embodiments, the radiation is administered prior to the checkpoint inhibitor and prior to Compound No. 14 or a pharmaceutically acceptable salt thereof. In some embodiments, the radiation is administered at least 40 hours before administration of the checkpoint inhibitor or Compound No. 14 or a pharmaceutically acceptable salt thereof. As used herein, the administration in "combination" of Compound No. 14 or a pharmaceutically acceptable salt thereof, a checkpoint inhibitor, radiation, and optionally one or more additional therapeutic agents refers not only to simultaneous or sequential administration of the agents and radiation, but also to the administration of the agents and radiation during a single treatment cycle, as understood by one skilled in the art. When Compound No. 14 or a pharmaceutically acceptable salt thereof is administered in combination with the checkpoint inhibitor, radiation, and optionally one or more additional therapeutic agents, a therapeutically effective amount of the combination is administered.

In some embodiments, Compound No. 14 and the checkpoint inhibitor are administered to a patient after radiation. In some embodiments, Compound No. 14 and the checkpoint inhibitor are administered to a patient who has previously undergone treatment with radiation. In some embodiments, Compound No. 14 and the checkpoint inhibitor are administered to a patient from 1 day to 3 months after the patient received treatment with radiation. In some embodiments, Compound No. 14 and the checkpoint inhibitor are administered to a patient from 1 day to 3 months, or from 1 day to 2 months, or from 1 day to 1 month, or from 1 day to 2 weeks, or from 1 day to 1 week after the patient received treatment with radiation.

The STING agonist may be administered by any method known to one skilled in the art. For example, in some embodiments, the STING agonist may be administered in the form of a pharmaceutical composition of the STING agonist and a pharmaceutically acceptable carrier, such as those described herein. In some embodiments, the pharmaceutical composition is suitable for oral administration. In some embodiments, the pharmaceutical composition is a tablet or a capsule that is suitable for oral administration. In some other embodiments, the pharmaceutical composition is a liquid dosage form suitable for oral administration. In some embodiments, the pharmaceutical composition is suitable for parenteral administration. In some embodiments, the pharmaceutical composition is suitable for intravenous administration. In some embodiments, the pharmaceutical composition is suitable for intravenous infusion. In some embodiments, the pharmaceutical composition is suitable for injection. In some embodiments, the pharmaceutical composition is suitable for intravenous injection. In some embodiments, the pharmaceutical composition is suitable for subcutaneous injection. In some embodiments, these compositions optionally further comprise one or more additional therapeutic agents.

The checkpoint inhibitor may be administered by any method known to one skilled in the art. In some embodiments, the checkpoint inhibitor is administered intravenously (i.v.). In some embodiments, the checkpoint inhibitor is administered subcutaneously (s.c.). In some embodiments, the checkpoint inhibitor is administered orally. For example, the checkpoint inhibitor may be administered in the form of a second composition, in some embodiments, a pharmaceutical composition of the checkpoint inhibitor and a pharmaceutically acceptable carrier, such as those described herein. In some aspects, the pharmaceutical composition is suitable for oral administration. In some embodiments, the pharmaceutical composition is a tablet or a capsule that is suitable for oral administration. In some other embodiments, the pharmaceutical composition is a liquid dosage form suitable for oral administration. In some embodiments, these compositions optionally further comprise one or more additional therapeutic agents.

In some embodiments, the checkpoint inhibitor may be administered orally, parenterally, by inhalation spray, topically, rectally, nasally, buccally, vaginally or via an implanted reservoir. The term "parenteral" as used herein includes subcutaneous, intravenous, intraperitoneal, intramuscular, intra-articular, intra-synovial, intrasternal, intrathecal, intrahepatic, intralesional and intracranial injection or infusion techniques. In some embodiments, the checkpoint inhibitor is administered orally, intravenously or subcutaneously. In some embodiments, the checkpoint inhibitor is administered orally. In some embodiments, the checkpoint inhibitor is administered intravenously. In some embodiments, the intravenous administration can be intravenous infusion or intravenous injection. In some embodiments, the checkpoint inhibitor is administered by an intravenous infusion. In some embodiments, the checkpoint inhibitor is administered by an intravenous injection. In some embodiments, the checkpoint inhibitor is administered by subcutaneous injection. In some embodiments, the checkpoint inhibitor is administered by intravenous infusion and then subsequently administered by subcutaneous injection. In another embodiment, the checkpoint inhibitor is coadministered with human hyaluronidase subcutaneously. These methods of administration may be designed to be short-acting, fast-releasing, or long-acting. Furthermore, the checkpoint inhibitor may be administered in a local rather than systemic means, such as administration (e.g., by injection) at a tumor site.

In some embodiments, the checkpoint inhibitor may also be administered by nasal aerosol or inhalation. The checkpoint inhibitor may be prepared according to techniques well known in the art and may be prepared as solutions in saline, employing benzyl alcohol or other suitable preservatives, absorption promoters to enhance bioavailability, fluorocarbons, and/or other conventional solubilizing or dispersing agents.

The amounts or suitable doses of the methods of this disclosure depends upon a number of factors, including the nature of the severity of the condition to be treated, the particular inhibitor, the route of administration and the age, weight, general health, and response of the individual patient. In some embodiments, the suitable dose level is one that achieves a therapeutic response as measured by tumor regression, or other standard measures of disease progression, progression free survival or overall survival. In some embodiments, the suitable dose level is one that achieves this therapeutic response and also minimizes any side effects associated with the administration of the therapeutic agent. The suitable dose levels may be ones that prolong the therapeutic response and/or prolong life.

It will be understood that a suitable dose of the STING agonist, the checkpoint inhibitor, and optionally one or more additional therapeutic agents may be taken at any time of the day or night. In some embodiments, a suitable dose of each agent is taken in the morning. In some other embodiments, a suitable dose of each agent is taken in the evening. In some embodiments, a suitable dose of each of the agents is taken both in the morning and the evening. It will be understood that a suitable dose of each agent may be taken with or without food. In some embodiments a suitable dose of an agent is taken with a meal. In some embodiments a suitable dose of an agent is taken while fasting.

In some embodiments, Compound No. 14 or a pharmaceutically acceptable salt thereof is administered on a daily schedule. In some embodiments, Compound No. 14 or a pharmaceutically acceptable salt thereof is administered every other day. In some embodiments, Compound No. 14 or a pharmaceutically acceptable salt thereof is administered once every three days. In some embodiments, Compound No. 14 or a pharmaceutically acceptable salt thereof is administered once every three days for three doses. In some embodiments, Compound No. 14 or a pharmaceutically acceptable salt thereof is administered on a twice-weekly schedule. In some embodiments, Compound No. 14 or a pharmaceutically acceptable salt thereof is administered on a three times a week schedule. In some embodiments, Compound No. 14 or a pharmaceutically acceptable salt thereof is administered on a weekly schedule. In some embodiments, Compound No. 14 or a pharmaceutically acceptable salt thereof is administered on a once every two weeks schedule.

In some embodiments, Compound No. 14 or a pharmaceutically acceptable salt thereof is administered once per day. In some embodiments, Compound No. 14 or a pharmaceutically acceptable salt thereof is administered twice per day. In some embodiments, Compound No. 14 or a pharmaceutically acceptable salt thereof is administered three times per day.

In some embodiments, Compound No. 14 or a pharmaceutically acceptable salt thereof is administered at least 3 times on alternate days within a 7-day cycle. In some embodiments, Compound No. 14 or a pharmaceutically acceptable salt thereof is administered on day 1 and day 4 of a 7-day cycle. In some embodiments, Compound No. 14 or a pharmaceutically acceptable salt thereof is administered on consecutive days in a 7-day cycle followed by an intermission. In some embodiments, Compound No. 14 or a pharmaceutically acceptable salt thereof is administered for 2 consecutive days followed by an intermission of 5 consecutive days for at least one 7-day cycle. In some embodiments, Compound No. 14 or a pharmaceutically acceptable salt thereof is administered for 3 consecutive days followed by an intermission of 4 consecutive days for at least one 7-day cycle. In some embodiments, Compound No. 14 or a pharmaceutically acceptable salt thereof is administered for 4 consecutive days followed by an intermission of 3 consecutive days for at least one 7-day cycle. In some embodiments, Compound No. 14 or a pharmaceutically acceptable salt thereof is administered for 5 consecutive days followed by an intermission of 2 consecutive days for at least one 7-day cycle. In some embodiments, there will be periods of rest between one or more of the 7-day treatment cycles. In some embodiments, there will be a 7-day rest between one or more of the 7-day treatment cycles.

The present description contemplates administration of the STING agonist for one or more treatment cycles, for example, 1, 2, 3, 4, 5, 6, or more, treatment cycles. In some embodiments, a treatment cycle is about 7 days to about 56 days, or more. In some embodiments, a treatment cycle is 7 days, 14 days, 21 days, 28 days, 35 days, 42 days, 49 days, or 56 days. In some embodiments, a treatment cycle is 21 days or 28 days. In some embodiments, there will be periods of rest within or between one or more of the treatment cycles. For example, in some embodiments, there will be a period of rest at the end of the treatment cycle. In some embodiments, there will be a period of rest between the second and third treatment cycle but not the first and second treatment cycle. In another embodiment, there might be a period of rest between the first and second treatment cycle but not the second and third treatment cycle. Dosing schedules include, for example, administering the STING agonist once during a treatment schedule, e.g., on day 1 of a 21 day cycle, twice during a treatment cycle, e.g., on days 1 and 15 of a 21 day cycle or on days 1 and 15 of a 28 day cycle, three times during a treatment cycle, e.g., on days 1, 8 and 15 of a 21 day cycle or on days 1, 8 and 15 of a 28 day cycle, and four times during a treatment cycle, e.g., on days 1, 4, 8, and 11 of a 21 day cycle or of on days 1, 4, 8, and 11 of a 28 day cycle. Other dosage schedules are encompassed by the present invention.

In some embodiments, Compound No. 14 or a pharmaceutically acceptable salt thereof is administered within a 21-day cycle. In some embodiments, Compound No. 14 or a pharmaceutically acceptable salt thereof is administered at least four times within a 21-day cycle. In some embodiments, Compound No. 14 or a pharmaceutically acceptable salt thereof is administered on day 1 within a 21-day cycle. In some embodiments, Compound No. 14 or a pharmaceutically acceptable salt thereof is administered on day 4 within a 21-day cycle. In some embodiments, Compound No. 14 or a pharmaceutically acceptable salt thereof is administered on day 8 within a 21-day cycle. In some embodiments, Compound No. 14 or a pharmaceutically acceptable salt thereof is administered on day 11 within a 21-day cycle. In some embodiments, Compound No. 14 or a pharmaceutically acceptable salt thereof is administered on days 1, 4, 8, and 11 within a 21-day cycle.

In some embodiments, Compound No. 14 or a pharmaceutically acceptable salt thereof is administered within a 21-day cycle. In some embodiments, Compound No. 14 or a pharmaceutically acceptable salt thereof is administered at least two times within a 21-day cycle. In some embodiments, Compound No. 14 or a pharmaceutically acceptable salt thereof is administered on day 1 within a 21-day cycle. In some embodiments, Compound No. 14 or a pharmaceutically acceptable salt thereof is administered on day 8 within a 21-day cycle. In some embodiments, Compound No. 14 or a pharmaceutically acceptable salt thereof is administered on days 1 and 8 within a 21-day cycle.

In some embodiments, Compound No. 14 or a pharmaceutically acceptable salt thereof is administered within a 21-day cycle. In some embodiments, Compound No. 14 or a pharmaceutically acceptable salt thereof is administered at least three times within a 21-day cycle. In some embodiments, Compound No. 14 or a pharmaceutically acceptable salt thereof is administered on day 1 within a 21-day cycle. In some embodiments, Compound No. 14 or a pharmaceutically acceptable salt thereof is administered on day 8 within a 21-day cycle. In some embodiments, Compound No. 14 or a pharmaceutically acceptable salt thereof is administered on day 15 within a 21-day cycle. In some embodiments, Compound No. 14 or a pharmaceutically acceptable salt thereof is administered on days 1, 8, and 15 within a 21-day cycle.

In some embodiments, Compound No. 14 or a pharmaceutically acceptable salt thereof is administered for a duration of 1 year or less. In some embodiments, Compound No. 14 or a pharmaceutically acceptable salt thereof is administered for a duration of 1 year or more.

In some embodiments, the amount of Compound No. 14 or a pharmaceutically acceptable salt thereof that is administered on each day of dosing is about 0.5 mg to about 1000 mg. In some embodiments, the amount of Compound No. 14 or a pharmaceutically acceptable salt thereof that is administered on each day of dosing is about 0.5 mg to about 300 mg. In some embodiments, the amount of Compound No. 14 or a pharmaceutically acceptable salt thereof that is administered on each day of dosing is about 1 mg to about 300 mg. In some embodiments, the amount of Compound No. 14 or a pharmaceutically acceptable salt thereof that is administered on each day of dosing is about 3 mg to about 300 mg. In some embodiments, the amount of Compound No. 14 or a pharmaceutically acceptable salt thereof that is administered on each day of dosing is about 0.5 mg to about 200 mg. In some embodiments, the amount of Compound No. 14 or a pharmaceutically acceptable salt thereof that is administered on each day of dosing is about 1 mg to about 200 mg. In some embodiments, the amount of Compound No. 14 or a pharmaceutically acceptable salt thereof that is administered on each day of dosing is about 10 mg to about 200 mg. In some embodiments, the amount of Compound No. 14 or a pharmaceutically acceptable salt thereof that is administered on each day of dosing is about 0.5 mg to about 100 mg. In some embodiments, the amount of Compound No. 14 or a pharmaceutically acceptable salt thereof that is administered on each day of dosing is about 0.5 mg to about 50 mg. In some embodiments, the amount of Compound No. 14 or a pharmaceutically acceptable salt thereof that is administered on each day of dosing is about 0.5 mg to about 10 mg. In some embodiments, the amount of Compound No. 14 or a pharmaceutically acceptable salt thereof that is administered on each day of dosing is about 0.5 mg to about 5 mg. In some embodiments, the amount of Compound No. 14 or a pharmaceutically acceptable salt thereof that is administered on each day of dosing is about 1 mg to about 3 mg. In some embodiments, the amount of Compound No. 14 or a pharmaceutically acceptable salt thereof that is administered on each day of dosing is about 2 mg to about 5 mg. In some embodiments, the amount of Compound No. 14 or a pharmaceutically acceptable salt thereof that is administered on each day of dosing is about 5 mg to about 10 mg. In some embodiments, the amount of Compound No. 14 or a pharmaceutically acceptable salt thereof that is administered on each day of dosing is about 5 mg to about 15 mg. In some embodiments, the amount of Compound No. 14 or a pharmaceutically acceptable salt thereof that is administered on each day of dosing is about 10 mg to about 20 mg. In some embodiments, the amount of Compound No. 14 or a pharmaceutically acceptable salt thereof that is administered on each day of dosing is about 15 mg to about 25 mg. In some embodiments, the amount of Compound No. 14 or a pharmaceutically acceptable salt thereof that is administered on each day of dosing is between about 20 mg to about 30 mg. In some embodiments, the amount of Compound No. 14 or a pharmaceutically acceptable salt thereof that is administered on each day of dosing is between about 25 mg to about 35 mg. In some embodiments, the amount of Compound No. 14 or a pharmaceutically acceptable salt thereof that is administered on each day of dosing is between about 30 mg to about 40 mg. In some embodiments, the amount of Compound No. 14 or a pharmaceutically acceptable salt thereof that is administered on each day of dosing is between about 35 mg to about 45 mg. In some embodiments, the amount of Compound No. 14 or a pharmaceutically acceptable salt thereof that is administered on each day of dosing is between about 40 mg to about 50 mg. In some embodiments, the amount of Compound No. 14 or a pharmaceutically acceptable salt thereof that is administered on each day of dosing is between about 55 mg to about 65 mg. In some embodiments, the amount of Compound No. 14 or a pharmaceutically acceptable salt thereof that is administered on each day of dosing is between about 50 mg to about 100 mg. In some embodiments, the amount of Compound No. 14 or a pharmaceutically acceptable salt thereof that is administered on each day of dosing is between about 90 mg to about 150 mg. In some embodiments, the amount of Compound No. 14 or a pharmaceutically acceptable salt thereof that is administered on each day of dosing is between about 140 mg to about 200 mg. In some embodiments, the amount of Compound No. 14 or a pharmaceutically acceptable salt thereof that is administered on each day of dosing is between about 190 mg to about 250 mg. In some embodiments, the amount of Compound No. 14 or a pharmaceutically acceptable salt thereof that is administered on each day of dosing is between about 240 mg to about 300 mg. In some embodiments, the amount of Compound No. 14 or a pharmaceutically acceptable salt thereof that is administered on each day of dosing is between about 290 mg to about 350 mg. In some embodiments, the amount of Compound No. 14 or a pharmaceutically acceptable salt thereof that is administered on each day of dosing is between about 340 mg to about 400 mg. In some embodiments, the amount of Compound No. 14 or a pharmaceutically acceptable salt thereof that is administered on each day of dosing is between about 390 mg to about 450 mg. In some embodiments, the amount of Compound No. 14 or a pharmaceutically acceptable salt thereof that is administered on each day of dosing is between about 440 mg to about 500 mg.

In some embodiments, the amount of Compound No. 14 or a pharmaceutically acceptable salt thereof that is administered on each day of dosing is about 0.5 mg. In some embodiments, the amount of Compound No. 14 or a pharmaceutically acceptable salt thereof that is administered on each day of dosing is about 1 mg. In some embodiments, the amount of Compound No. 14 or a pharmaceutically acceptable salt thereof that is administered on each day of dosing is about 2 mg. In some embodiments, the amount of Compound No. 14 or a pharmaceutically acceptable salt thereof that is administered on each day of dosing is about 3 mg. In some embodiments, the amount of Compound No. 14 or a pharmaceutically acceptable salt thereof that is administered on each day of dosing is about 4 mg. In some embodiments, the amount of Compound No. 14 or a pharmaceutically acceptable salt thereof that is administered on each day of dosing is about 6 mg. In some embodiments, the amount of Compound No. 14 or a pharmaceutically acceptable salt thereof that is administered on each day of dosing is about 8 mg. In some embodiments, the amount of Compound No. 14 or a pharmaceutically acceptable salt thereof that is administered on each day of dosing is about 10 mg. In some embodiments, the amount of Compound No. 14 or a pharmaceutically acceptable salt thereof that is administered on each day of dosing is about 12 mg. In some embodiments, the amount of Compound No. 14 or a pharmaceutically acceptable salt thereof that is administered on each day of dosing is about 16 mg. In some embodiments, the amount of Compound No. 14 or a pharmaceutically acceptable salt thereof that is administered on each day of dosing is about 20 mg. In some embodiments, the amount of Compound No. 14 or a pharmaceutically acceptable salt thereof that is administered on each day of dosing is about 30 mg. In some embodiments, the amount of Compound No. 14 or a pharmaceutically acceptable salt thereof that is administered on each day of dosing is about 40 mg. In some embodiments, the amount of Compound No. 14 or a pharmaceutically acceptable salt thereof that is administered on each day of dosing is about 50 mg. In some embodiments, the amount of Compound No. 14 or a pharmaceutically acceptable salt thereof that is administered on each day of dosing is about 60 mg. In some embodiments, the amount of Compound No. 14 or a pharmaceutically acceptable salt thereof that is administered on each day of dosing is about 70 mg. In some embodiments, the amount of Compound No. 14 or a pharmaceutically acceptable salt thereof that is administered on each day of dosing is about 80 mg. In some embodiments, the amount of Compound No. 14 or a pharmaceutically acceptable salt thereof that is administered on each day of dosing is about 90 mg. In some embodiments, the amount of Compound No. 14 or a pharmaceutically acceptable salt thereof that is administered on each day of dosing is about 100 mg. In some embodiments, the amount of Compound No. 14 or a pharmaceutically acceptable salt thereof that is administered on each day of dosing is about 150 mg. In some embodiments, the amount of Compound No. 14 or a pharmaceutically acceptable salt thereof that is administered on each day of dosing is about 200 mg. In some embodiments, the amount of Compound No. 14 or a pharmaceutically acceptable salt thereof that is administered on each day of dosing is about 250 mg. In some embodiments, the amount of Compound No. 14 or a pharmaceutically acceptable salt thereof that is administered on each day of dosing is about 300 mg. In some embodiments, the amount of Compound No. 14 or a pharmaceutically acceptable salt thereof that is administered on each day of dosing is about 350 mg. In some embodiments, the amount of Compound No. 14 or a pharmaceutically acceptable salt thereof that is administered on each day of dosing is about 400 mg. In some embodiments, the amount of Compound No. 14 or a pharmaceutically acceptable salt thereof that is administered on each day of dosing is about 450 mg. In some embodiments, the amount of Compound No. 14 or a pharmaceutically acceptable salt thereof that is administered on each day of dosing is about 500 mg. All dosing amounts refer to the amount of Compound No. 14 administered, and do not include the weight amount of any pharmaceutically acceptable salt.

In some embodiments, the amount of Compound No. 14 or a pharmaceutically acceptable salt thereof that is administered on each day of dosing is from about 0.05 mg to about 3.5 mg. In some embodiments, the amount of Compound No. 14 or a pharmaceutically acceptable salt thereof that is administered on each day of dosing is from about 0.1 mg to about 3.5 mg. In some embodiments, the amount of Compound No. 14 or a pharmaceutically acceptable salt thereof that is administered on each day of dosing is from about 0.2 mg to about 3.5 mg. In some embodiments, the amount of Compound No. 14 or a pharmaceutically acceptable salt thereof that is administered on each day of dosing is from about 0.2 mg to about 2.5 mg. In some embodiments, the amount of Compound No. 14 or a pharmaceutically acceptable salt thereof that is administered on each day of dosing is from about 0.05 mg to about 1.2 mg. In some embodiments, the amount of Compound No. 14 or a pharmaceutically acceptable salt thereof that is administered on each day of dosing is from about 0.1 mg to about 1.2 mg. In some embodiments, the amount of Compound No. 14 or a pharmaceutically acceptable salt thereof that is administered on each day of dosing is from about 0.2 mg to about 1.2 mg. In some embodiments, the amount of Compound No. 14 or a pharmaceutically acceptable salt thereof that is administered on each day of dosing is about 0.05 mg. In some embodiments, the amount of Compound No. 14 or a pharmaceutically acceptable salt thereof that is administered on each day of dosing is about 0.1 mg. In some embodiments, the amount of Compound No. 14 or a pharmaceutically acceptable salt thereof that is administered on each day of dosing is about 0.2 mg. In some embodiments, the amount of Compound No. 14 or a pharmaceutically acceptable salt thereof that is administered on each day of dosing is about 0.4 mg. In some embodiments, the amount of Compound No. 14 or a pharmaceutically acceptable salt thereof that is administered on each day of dosing is about 0.8 mg. In some embodiments, the amount of Compound No. 14 or a pharmaceutically acceptable salt thereof that is administered on each day of dosing is about 1.2 mg. In some embodiments, the amount of Compound No. 14 or a pharmaceutically acceptable salt thereof that is administered on each day of dosing is about 1.6 mg. In some embodiments, the amount of Compound No. 14 or a pharmaceutically acceptable salt thereof that is administered on each day of dosing is about 1.8 mg. In some embodiments, the amount of Compound No. 14 or a pharmaceutically acceptable salt thereof that is administered on each day of dosing is about 2.0 mg. In some embodiments, the amount of Compound No. 14 or a pharmaceutically acceptable salt thereof that is administered on each day of dosing is about 2.25 mg. In some embodiments, the amount of Compound No. 14 or a pharmaceutically acceptable salt thereof that is administered on each day of dosing is about 2.5 mg. In some embodiments, the amount of Compound No. 14 or a pharmaceutically acceptable salt thereof that is administered on each day of dosing is about 2.8 mg. In some embodiments, the amount of Compound No. 14 or a pharmaceutically acceptable salt thereof that is administered on each day of dosing is about 3.0 mg. In some embodiments, the amount of Compound No. 14 or a pharmaceutically acceptable salt thereof that is administered on each day of dosing is about 3.5 mg.

In some embodiments, the amount of Compound No. 14 or a pharmaceutically acceptable salt thereof that is administered on each day of dosing is about 0.01 mg/kg to about 100 mg/kg. In some embodiments, the amount of Compound No. 14 or a pharmaceutically acceptable salt thereof that is administered on each day of dosing is about 0.01 mg/kg to about 50 mg/kg. In some embodiments, the amount of Compound No. 14 or a pharmaceutically acceptable salt thereof that is administered on each day of dosing is about 0.01 mg/kg to about 20 mg/kg.

In some embodiments, the amount of Compound No. 14 or a pharmaceutically acceptable salt thereof that is administered on each day of dosing is about 0.01 mg/kg. In some embodiments, the amount of Compound No. 14 or a pharmaceutically acceptable salt thereof that is administered on each day of dosing is about 0.05 mg/kg. In some embodiments, the amount of Compound No. 14 or a pharmaceutically acceptable salt thereof that is administered on each day of dosing is about 0.1 mg/kg. In some embodiments, the amount of Compound No. 14 or a pharmaceutically acceptable salt thereof that is administered on each day of dosing is about 1 mg/kg. In some embodiments, the amount of Compound No. 14 or a pharmaceutically acceptable salt thereof that is administered on each day of dosing is about 2 mg/kg. In some embodiments, the amount of Compound No. 14 or a pharmaceutically acceptable salt thereof that is administered on each day of dosing is about 4 mg/kg. In some embodiments, the amount of Compound No. 14 or a pharmaceutically acceptable salt thereof that is administered on each day of dosing is about 6 mg/kg. In some embodiments, the amount of Compound No. 14 or a pharmaceutically acceptable salt thereof that is administered on each day of dosing is about 8 mg/kg. In some embodiments, the amount of Compound No. 14 or a pharmaceutically acceptable salt thereof that is administered on each day of dosing is about 10 mg/kg. In some embodiments, the amount of Compound No. 14 or a pharmaceutically acceptable salt thereof that is administered on each day of dosing is about 12 mg/kg. In some embodiments, the amount of Compound No. 14 or a pharmaceutically acceptable salt thereof that is administered on each day of dosing is about 14 mg/kg. In some embodiments, the amount of Compound No. 14 or a pharmaceutically acceptable salt thereof that is administered on each day of dosing is about 16 mg/kg. In some embodiments, the amount of Compound No. 14 or a pharmaceutically acceptable salt thereof that is administered on each day of dosing is about 18 mg/kg. In some embodiments, the amount of Compound No. 14 or a pharmaceutically acceptable salt thereof that is administered on each day of dosing is about 20 mg/kg. In some embodiments, the amount of Compound No. 14 or a pharmaceutically acceptable salt thereof that is administered on each day of dosing is about 30 mg/kg. In some embodiments, the amount of Compound No. 14 or a pharmaceutically acceptable salt thereof that is administered on each day of dosing is about 40 mg/kg. In some embodiments, the amount of Compound No. 14 or a pharmaceutically acceptable salt thereof that is administered on each day of dosing is about 50 mg/kg. In some embodiments, the amount of Compound No. 14 or a pharmaceutically acceptable salt thereof that is administered on each day of dosing is about 60 mg/kg. In some embodiments, the amount of Compound No. 14 or a pharmaceutically acceptable salt thereof that is administered on each day of dosing is about 70 mg/kg. In some embodiments, the amount of Compound No. 14 or a pharmaceutically acceptable salt thereof that is administered on each day of dosing is about 80 mg/kg. In some embodiments, the amount of Compound No. 14 or a pharmaceutically acceptable salt thereof that is administered on each day of dosing is about 90 mg/kg. In some embodiments, the amount of Compound No. 14 or a pharmaceutically acceptable salt thereof that is administered on each day of dosing is about 100 mg/kg.

In some embodiments, the checkpoint inhibitor is administered on a daily schedule. In some embodiments, the checkpoint inhibitor is administered every other day. In some embodiments, the checkpoint inhibitor is administered once every three days. In some embodiments, the checkpoint inhibitor is administered on a twice-weekly schedule. In some embodiments, the checkpoint inhibitor is administered on a three times a week schedule. In some embodiments, the checkpoint inhibitor is administered on a weekly schedule. In some embodiments, the checkpoint inhibitor is administered on a once every two weeks schedule. In some embodiments, the checkpoint inhibitor is administered on a once every three weeks schedule. In some embodiments, the checkpoint inhibitor is administered on a once every four weeks schedule. In some embodiments, the checkpoint inhibitor is administered on a once every eight weeks schedule. In some embodiments, the checkpoint inhibitor is administered on a once every twelve weeks schedule.

In some embodiments, the checkpoint inhibitor is administered at least 3 times on alternate days within a 7-day cycle. In some embodiments, the checkpoint inhibitor is administered on day 1 of a treatment cycle. In some embodiments, the checkpoint inhibitor is administered on day 1 and day 4 of a 7-day cycle. In some embodiments, the checkpoint inhibitor is administered on consecutive days in a 7-day cycle followed by an intermission. In some embodiments, the checkpoint inhibitor is administered for 2 consecutive days followed by an intermission of 5 consecutive days for at least one 7-day cycle. In some embodiments, the checkpoint inhibitor is administered for 3 consecutive days followed by an intermission of 4 consecutive days for at least one 7-day cycle. In some embodiments, the checkpoint inhibitor is administered for 4 consecutive days followed by an intermission of 3 consecutive days for at least one 7-day cycle. In some embodiments, the checkpoint inhibitor is administered for 5 consecutive days followed by an intermission of 2 consecutive days for at least one 7-day cycle.

In some embodiments, the checkpoint inhibitor is administered on day 1 of a 21-day treatment cycle. In some embodiments, the checkpoint inhibitor is administered on day 2 of a 21-day treatment cycle. In some embodiments, the checkpoint inhibitor is administered on day 2 of a first 21-day treatment cycle and on day 1 of each subsequent 21-day treatment cycle.

The present description contemplates administration of the checkpoint inhibitor for one or more treatment cycles, for example, 1, 2, 3, 4, 5, 6, or more, treatment cycles. In some embodiments, a treatment cycle is about 7 days to about 84 days, or more. In some embodiments, a treatment cycle is 7 days, 14 days, 21 days, 28 days, 35 days, 42 days, 49 days, 56 days, or 84 days. In some embodiments, a treatment cycle is 21 days or 28 days. In some embodiments, there will be periods of rest within or between one or more of the treatment cycles. For example, in some embodiments, there will be a period of rest at the end of the treatment cycle. In some embodiments, there will be a period of rest between the second and third treatment cycle but not the first and second treatment cycle. In another embodiment, there might be a period of rest between the first and second treatment cycle but not the second and third treatment cycle. Dosing schedules include, for example, administering the checkpoint inhibitor once during a treatment schedule, e.g., on day 1 of a 21 day cycle, twice during a treatment cycle, e.g., on days 1 and 15 of a 21 day cycle or on days 1 and 15 of a 28 day cycle, three times during a treatment cycle, e.g., on days 1, 8 and 15 of a 21 day cycle or on days 1, 8 and 15 of a 28 day cycle, and four times during a treatment cycle, e.g., on days 1, 4, 8, and 11 of a 21 day cycle or of on days 1, 4, 8, and 11 of a 28 day cycle. Other dosage schedules are encompassed by the present invention.

In some embodiments, the checkpoint inhibitor is administered by subcutaneous injection. In some embodiments, the checkpoint inhibitor is administered by intravenous infusion followed by one or more subsequent subcutaneous injections. In some embodiments, the intravenous infusion and one or more subsequent subcutaneous injections are administered according to the dosing schedules and methods disclosed herein.

In some embodiments, both Compound No. 14 and the checkpoint inhibitor are administered on day 1 of a 21-day treatment cycle. In some embodiments, Compound No. 14 is administered first on day 1 of a 21-day treatment cycle followed by the checkpoint inhibitor. In some embodiments, Compound No. 14 is administered on day 1 of a 21-day treatment cycle and the checkpoint inhibitor is administered on day 1 of a 21-day treatment cycle 1 hour after administration Compound No. 14. In some embodiments, the checkpoint inhibitor is administered first on day 1 of a 21-day treatment cycle followed by Compound No. 14.

In some embodiments, the radiation is administered first in the treatment cycle, the checkpoint inhibitor is administered second in the treatment cycle, and Compound No. 14 is administered third in the treatment cycle. In some embodiments, the radiation is administered first in the treatment cycle, Compound No. 14 is administered second in the treatment cycle, and the checkpoint inhibitor is administered third in the treatment cycle. In some embodiments, the radiation is administered first in the treatment cycle followed by simultaneous administration of Compound No. 14 and the checkpoint inhibitor. In some embodiments, the radiation is administered first in the treatment cycle followed by sequential administration of Compound No. 14 and the checkpoint inhibitor. In some embodiments, the radiation is administered before the treatment cycle, and the checkpoint inhibitor and Compound No. 14 are administered on day 1 of the treatment cycle. In some embodiments, the radiation is administered before the treatment cycle, Compound No. 14 is administered on day 1 of the treatment cycle, and the checkpoint inhibitor is administered once every three weeks. In some embodiments, the radiation is administered before the 21 day treatment cycle, Compound No. 14 is administered on days 1, 8, and 15 of the 21 day treatment cycle, and the checkpoint inhibitor is administered once every three weeks.

In some embodiments, Compound No. 14 is administered as a 60 ±10-minute intravenous infusion.

In some embodiments, the amount of the anti-PD-1 antibody that is administered on each day of dosing is about 0.5 mg to about 1000 mg. In some embodiments, the amount of the anti-PD-1 antibody that is administered on each day of dosing is about 0.5 mg to about 900 mg. In some embodiments, the amount of the anti-PD-1 antibody that is administered on each day of dosing is about 0.5 mg to about 800 mg. In some embodiments, the amount of the anti-PD-1 antibody that is administered on each day of dosing is about 0.5 mg to about 700 mg. In some embodiments, the amount of the anti-PD-1 antibody that is administered on each day of dosing is about 0.5 mg to about 600 mg. In some embodiments, the amount of the anti-PD-1 antibody that is administered on each day of dosing is about 0.5 mg to about 500 mg. In some embodiments, the amount of the anti-PD-1 antibody that is administered on each day of dosing is about 1 mg to about 500 mg. In some embodiments, the amount of the anti-PD-1 antibody that is administered on each day of dosing is about 10 mg to about 500 mg. In some embodiments, the amount of the anti-PD-1 antibody that is administered on each day of dosing is about 50 mg to about 500 mg. In some embodiments, the amount of the anti-PD-1 antibody that is administered on each day of dosing is about 100 mg to about 500 mg. In some embodiments, the amount of the anti-PD-1 antibody that is administered on each day of dosing is about 150 mg to about 500 mg. In some embodiments, the amount of the anti-PD-1 antibody that is administered on each day of dosing is about 200 mg to about 500 mg. In some embodiments, the amount of the anti-PD-1 antibody that is administered on each day of dosing is about 220 mg to about 500 mg. In some embodiments, the amount of the anti-PD-1 antibody that is administered on each day of dosing is about 240 mg to about 500 mg. In some embodiments, the amount of the anti-PD-1 antibody that is administered on each day of dosing is about 260 mg to about 500 mg. In some embodiments, the amount of the anti-PD-1 antibody that is administered on each day of dosing is about 280 mg to about 500 mg. In some embodiments, the amount of the anti-PD-1 antibody that is administered on each day of dosing is about 300 mg to about 500 mg. In some embodiments, the amount of the anti-PD-1 antibody that is administered on each day of dosing is about 320 mg to about 500 mg. In some embodiments, the amount of the anti-PD-1 antibody that is administered on each day of dosing is about 340 mg to about 500 mg. In some embodiments, the amount of the anti-PD-1 antibody that is administered on each day of dosing is about 360 mg to about 500 mg. In some embodiments, the amount of the anti-PD-1 antibody that is administered on each day of dosing is about 380 mg to about 500 mg. In some embodiments, the amount of the anti-PD-1 antibody that is administered on each day of dosing is about 400 mg to about 500 mg. In some embodiments, the amount of the anti-PD-1 antibody that is administered on each day of dosing is about 200 mg to about 480 mg. In some embodiments, the amount of the anti-PD-1 antibody that is administered on each day of dosing is about 200 mg to about 460 mg. In some embodiments, the amount of the anti-PD-1 antibody that is administered on each day of dosing is about 200 mg to about 440 mg. In some embodiments, the amount of the anti-PD-1 antibody that is administered on each day of dosing is about 200 mg to about 420 mg. In some embodiments, the amount of the anti-PD-1 antibody that is administered on each day of dosing is about 200 mg to about 400 mg. In some embodiments, the amount of the anti-PD-1 antibody that is administered on each day of dosing is about 200 mg to about 380 mg. In some embodiments, the amount of the anti-PD-1 antibody that is administered on each day of dosing is about 200 mg to about 360 mg. In some embodiments, the amount of the anti-PD-1 antibody that is administered on each day of dosing is about 200 mg to about 340 mg. In some embodiments, the amount of the anti-PD-1 antibody that is administered on each day of dosing is about 200 mg to about 320 mg. In some embodiments, the amount of the anti-PD-1 antibody that is administered on each day of dosing is about 200 mg to about 300 mg.

In some embodiments, the amount of the anti-PD-1 antibody that is administered on each day of dosing is about 100 mg. In some embodiments, the amount of the anti-PD-1 antibody that is administered on each day of dosing is about 120 mg. In some embodiments, the amount of the anti-PD-1 antibody that is administered on each day of dosing is about 140 mg. In some embodiments, the amount of the anti-PD-1 antibody that is administered on each day of dosing is about 160 mg. In some embodiments, the amount of the anti-PD-1 antibody that is administered on each day of dosing is about 180 mg. In some embodiments, the amount of the anti-PD-1 antibody that is administered on each day of dosing is about 200 mg. In some embodiments, the amount of the anti-PD-1 antibody that is administered on each day of dosing is about 220 mg. In some embodiments, the amount of the anti-PD-1 antibody that is administered on each day of dosing is about 240 mg. In some embodiments, the amount of the anti-PD-1 antibody that is administered on each day of dosing is about 260 mg. In some embodiments, the amount of the anti-PD-1 antibody that is administered on each day of dosing is about 280 mg. In some embodiments, the amount of the anti-PD-1 antibody that is administered on each day of dosing is about 300 mg. In some embodiments, the amount of the anti-PD-1 antibody that is administered on each day of dosing is about 320 mg. In some embodiments, the amount of the anti-PD-1 antibody that is administered on each day of dosing is about 340 mg. In some embodiments, the amount of the anti-PD-1 antibody that is administered on each day of dosing is about 360 mg. In some embodiments, the amount of the anti-PD-1 antibody that is administered on each day of dosing is about 380 mg. In some embodiments, the amount of the anti-PD-1 antibody that is administered on each day of dosing is about 400 mg. In some embodiments, the amount of the anti-PD-1 antibody that is administered on each day of dosing is about 420 mg. In some embodiments, the amount of the anti-PD-1 antibody that is administered on each day of dosing is about 440 mg. In some embodiments, the amount of the anti-PD-1 antibody that is administered on each day of dosing is about 460 mg. In some embodiments, the amount of the anti-PD-1 antibody that is administered on each day of dosing is about 480 mg. In some embodiments, the amount of the anti-PD-1 antibody that is administered on each day of dosing is about 500 mg. In some embodiments, the amount of the anti-PD-1 antibody that is administered on each day of dosing is about 600 mg. In some embodiments, the amount of the anti-PD-1 antibody that is administered on each day of dosing is about 700 mg. In some embodiments, the amount of the anti-PD-1 antibody that is administered on each day of dosing is about 800 mg. In some embodiments, the amount of the anti-PD-1 antibody that is administered on each day of dosing is about 900 mg. In some embodiments, the amount of the anti-PD-1 antibody that is administered on each day of dosing is about 1000 mg.

In some embodiments, the amount of the anti-PD-1 antibody that is administered on each day of dosing is about 200 mg.

In some embodiments, the amount of the anti-PD-1 antibody that is administered on each day of dosing is about 0.5 mg/kg to about 10 mg/kg. In some embodiments, the amount of the anti-PD-1 antibody that is administered on each day of dosing is about 0.5 mg/kg to about 7.5 mg/kg. In some embodiments, the amount of the anti-PD-1 antibody that is administered on each day of dosing is about 0.5 mg/kg to about 5 mg/kg. In some embodiments, the amount of the anti-PD-1 antibody that is administered on each day of dosing is about 1 mg/kg to about 4 mg/kg. In some embodiments, the amount of the anti-PD-1 antibody that is administered on each day of dosing is about 1 mg/kg to about 3 mg/kg. In some embodiments, the amount of the anti-PD-1 antibody that is administered on each day of dosing is about 0.5 mg/kg. In some embodiments, the amount of the anti-PD-1 antibody that is administered on each day of dosing is about 1 mg/kg. In some embodiments, the amount of the anti-PD-1 antibody that is administered on each day of dosing is about 1.5 mg/kg. In some embodiments, the amount of the anti-PD-1 antibody that is administered on each day of dosing is about 2 mg/kg. In some embodiments, the amount of the anti-PD-1 antibody that is administered on each day of dosing is about 2.5 mg/kg. In some embodiments, the amount of the anti-PD-1 antibody that is administered on each day of dosing is about 3 mg/kg. In some embodiments, the amount of the anti-PD-1 antibody that is administered on each day of dosing is about 3.5 mg/kg. In some embodiments, the amount of the anti-PD-1 antibody that is administered on each day of dosing is about 4 mg/kg. In some embodiments, the amount of the anti-PD-1 antibody that is administered on each day of dosing is about 4.5 mg/kg. In some embodiments, the amount of the anti-PD-1 antibody that is administered on each day of dosing is about 5 mg/kg. In some embodiments, the amount of the anti-PD-1 antibody that is administered on each day of dosing is about 7.5 mg/kg. In some embodiments, the amount of the anti-PD-1 antibody that is administered on each day of dosing is about 10 mg/kg.

In some embodiments, the anti-PD-1 antibody is nivolumab, or a pharmaceutically acceptable salt thereof. In some embodiments, the anti-PD-1 antibody is pembrolizumab, or a pharmaceutically acceptable salt thereof. In some embodiments, the anti-PD-1 antibody is cemiplimab, or a pharmaceutically acceptable salt thereof.

In some embodiments, the administration of nivolumab, pembrolizumab, and cemiplimab is in accordance with its prescribing information as approved by the health authorities, such as those issued by the FDA, or the EMA

In some embodiments, the anti-PD-1 antibody is pembrolizumab, or a pharmaceutically acceptable salt thereof. In some embodiments, the amount of the pembrolizumab that is administered on each day of dosing is about 200 mg. In some embodiments, the pembrolizumab is administered on day 1 of a 21 day cycle in an amount of 200 mg. In some embodiments, the pembrolizumab is administered once every three weeks in an amount of 200 mg.

In some embodiments, the anti-PD-1 antibody is pembrolizumab, or a pharmaceutically acceptable salt thereof, and the pembrolizumab is administered in combination with Compound No. 14 and radiation. In some embodiments, the pembrolizumab is administered on day 1 of a 21-day cycle, Compound No. 14 is administered on days 1, 8, and 15 of a 21-day cycle, and the radiation is administered on day 1 of a 21 day cycle or before a 21 day cycle. In some embodiments, the pembrolizumab is administered on day 1 of a 21-day cycle, Compound No. 14 is administered on days 1, 8, and 15 of a 21-day cycle, and the radiation is administered between day -8 and day -1 of a 21 day cycle. In some embodiments, the pembrolizumab is administered on day 1 of a 21-day cycle, Compound No. 14 is administered on days 1, 8, and 15 of a 21-day cycle, and the radiation is administered between day -7 and day -1 of a 21 day cycle. In some embodiments, the pembrolizumab is administered on day 1 of a 21-day cycle, Compound No. 14 is administered on days 1, 8, and 15 of a 21-day cycle, and the radiation is administered at least 40 hours before administration of the pembrolizumab or Compound No. 14. In some embodiments, the pembrolizumab is administered in an amount of 200 mg on day 1 of a 21-day cycle, Compound No. 14 is administered in an amount of 0.1 mg on days 1, 8, and 15 of a 21-day cycle, and the radiation is administered at a fraction dose of about 5 Gy to about 20 Gy at least 40 hours before administration of the checkpoint inhibitor or Compound No. 14. In some embodiments, the pembrolizumab is administered in an amount of 200 mg on day 1 of a 21-day cycle, Compound No. 14 is administered in an amount of 0.2 mg on days 1, 8, and 15 of a 21-day cycle, and the radiation is administered at a fraction dose of about 5 Gy to about 20 Gy at least 40 hours before administration of the checkpoint inhibitor or Compound No. 14. In some embodiments, the pembrolizumab is administered in an amount of 200 mg on day 1 of a 21-day cycle, Compound No. 14 is administered in an amount of 0.2 mg or higher on days 1, 8, and 15 of a 21-day cycle, and the radiation is administered at a fraction dose of about 5 Gy to about 20 Gy at least 40 hours before administration of the checkpoint inhibitor or Compound No. 14. In some embodiments, the pembrolizumab is administered in an amount of 200 mg on day 1 of a 21-day cycle, Compound No. 14 is administered in an amount of from 0.1 mg to 3.5 mg on days 1, 8, and 15 of a 21-day cycle, and the radiation is administered at a fraction dose of about 5 Gy to about 20 Gy at least 40 hours before administration of the checkpoint inhibitor or Compound No. 14. In some embodiments, the pembrolizumab is administered in an amount of 200 mg on day 1 of a 21-day cycle, Compound No. 14 is administered in an amount of from 0.1 mg to 1.2 mg on days 1, 8, and 15 of a 21-day cycle, and the radiation is administered at a fraction dose of about 5 Gy to about 20 Gy at least 40 hours before administration of the checkpoint inhibitor or Compound No. 14. In some embodiments, the pembrolizumab is administered in an amount of 200 mg on day 1 of a 21-day cycle, Compound No. 14 is administered in an amount of from 0.2 mg to 3.5 mg on days 1, 8, and 15 of a 21-day cycle, and the radiation is administered at a fraction dose of about 5 Gy to about 20 Gy at least 40 hours before administration of the checkpoint inhibitor or Compound No. 14. In some embodiments, the pembrolizumab is administered in an amount of 200 mg on day 1 of a 21-day cycle, Compound No. 14 is administered in an amount of from 0.2 mg to 1.2 mg on days 1, 8, and 15 of a 21-day cycle, and the radiation is administered at a fraction dose of about 5 Gy to about 20 Gy at least 40 hours before administration of the checkpoint inhibitor or Compound No. 14. In some embodiments, the pembrolizumab is administered in an amount of 200 mg on day 1 of a 21-day cycle, Compound No. 14 is administered in an amount of 0.1 mg, 0.2 mg, 0.4 mg, 0.8 mg, 1.2 mg, 1.6 mg, 2.0 mg, 2.5 mg, 3.0 mg, or 3.5 mg on days 1, 8, and 15 of a 21-day cycle, and the radiation is administered at a fraction dose of about 5 Gy to about 20 Gy at least 40 hours before administration of the checkpoint inhibitor or Compound No. 14.

In some embodiments, the radiation is particle radiation.

In some embodiments, the radiation is administered by external beam radiation.

In some embodiments, the radiation is administered between day -15 and day 2 of a treatment cycle. In some embodiments, the radiation is administered between day -10 and day 1 of a treatment cycle. In some embodiments, the radiation is administered between day -10 and day 0 of a treatment cycle. In some embodiments, the radiation is administered between day -10 and day -1 of a treatment cycle. In some embodiments, the radiation is administered between day -9 and day -1 of a treatment cycle. In some embodiments, the radiation is administered between day -8 and day -1 of a treatment cycle. In some embodiments, the radiation is administered between day -7 and day -1 of a treatment cycle. In some embodiments, the treatment cycle is 21 days.

In some embodiments, the radiation is administered at least 5 hours before administration of the checkpoint inhibitor or Compound No. 14. In some embodiments, the radiation is administered at least 10 hours before administration of the checkpoint inhibitor or Compound No. 14. In some embodiments, the radiation is administered at least 20 hours before administration of the checkpoint inhibitor or Compound No. 14. In some embodiments, the radiation is administered at least 40 hours before administration of the checkpoint inhibitor or Compound No. 14. In some embodiments, the radiation is administered at least 80 hours before administration of the checkpoint inhibitor or Compound No. 14.

In some embodiments, the radiation is administered on each of days 1-5 of each week repeated for 2 to 8 weeks. In some embodiments, the radiation is administered on each of days 1-5 of each week repeated for 6 to 8 weeks. In some embodiments, the radiation is administered on each of days 1-5 of each week repeated for 2 weeks. In some embodiments, the radiation is administered on each of days 1-5 of each week repeated for 3 weeks. In some embodiments, the radiation is administered on each of days 1-5 of each week repeated for 4 weeks. In some embodiments, the radiation is administered on each of days 1-5 of each week repeated for 5 weeks. In some embodiments, the radiation is administered on each of days 1-5 of each week repeated for 6 weeks. In some embodiments, the radiation is administered on each of days 1-5 of each week repeated for 7 weeks. In some embodiments, the radiation is administered on each of days 1-5 of each week repeated for 8 weeks.

In some embodiments, the radiation is administered on any two of days 1-5 of each week repeated for 5 to 8 weeks. In some embodiments, the radiation is administered on any two of days 1-5 of each week repeated for 6 to 8 weeks. In some embodiments, the radiation is administered on any two of days 1-5 of each week repeated for 5 weeks. In some embodiments, the radiation is administered on any two of days 1-5 of each week repeated for 6 weeks. In some embodiments, the radiation is administered on any two of days 1-5 of each week repeated for 7 weeks. In some embodiments, the radiation is administered on any two of days 1-5 of each week repeated for 8 weeks.

In some embodiments, the radiation is administered at a fraction dose of about 1 Gy to about 100 Gy. In some embodiments, the radiation is administered at a fraction dose of about 1 Gy to about 50 Gy. In some embodiments, the radiation is administered at a fraction dose of about 1 Gy to about 20 Gy. In some embodiments, the radiation is administered at a fraction dose of about 5 Gy to about 20 Gy. In some embodiments, the radiation is administered at a fraction dose of about 6 Gy to about 18 Gy. In some embodiments, the radiation is administered at a fraction dose of about 8 Gy to about 16 Gy. In some embodiments, the radiation is administered at a fraction dose of about 5 Gy to about 10 Gy. In some embodiments, the radiation is administered at a fraction dose of about 10 Gy to about 15 Gy. In some embodiments, the radiation is administered at a fraction dose of about 15 Gy to about 20 Gy. In some embodiments, the radiation is administered at a fraction dose of about 1 Gy. In some embodiments, the radiation is administered at a fraction dose of about 2 Gy. In some embodiments, the radiation is administered at a fraction dose of about 3 Gy. In some embodiments, the radiation is administered at a fraction dose of about 4 Gy. In some embodiments, the radiation is administered at a fraction dose of about 5 Gy. In some embodiments, the radiation is administered at a fraction dose of about 6 Gy. In some embodiments, the radiation is administered at a fraction dose of about 7 Gy. In some embodiments, the radiation is administered at a fraction dose of about 8 Gy. In some embodiments, the radiation is administered at a fraction dose of about 9 Gy. In some embodiments, the radiation is administered at a fraction dose of about 10 Gy. In some embodiments, the radiation is administered at a fraction dose of about 11 Gy. In some embodiments, the radiation is administered at a fraction dose of about 12 Gy. In some embodiments, the radiation is administered at a fraction dose of about 13 Gy. In some embodiments, the radiation is administered at a fraction dose of about 14 Gy. In some embodiments, the radiation is administered at a fraction dose of about 15 Gy. In some embodiments, the radiation is administered at a fraction dose of about 16 Gy. In some embodiments, the radiation is administered at a fraction dose of about 17 Gy. In some embodiments, the radiation is administered at a fraction dose of about 18 Gy. In some embodiments, the radiation is administered at a fraction dose of about 19 Gy. In some embodiments, the radiation is administered at a fraction dose of about 20 Gy. In some embodiments, the radiation is administered at a fraction dose of about 8 Gy or about 16 Gy.

In some embodiments, the radiation is administered in fractions. In some embodiments, the radiation is administered in from 1 to 10 fractions. In some embodiments, the radiation is administered in from 1 to 5 fractions. In some embodiments, the radiation is administered in 1 fraction, or in 2 fractions, or in 3 fractions, or in 4 fractions, or in 5 fractions. In some embodiments, the radiation is administered in 1 fraction or in 3 fractions.

In some embodiments, the radiation is administered at a fraction dose of about 1-5 Gy for 1-3 fractions. In some embodiments, the radiation is administered at a fraction dose of about 5-10 Gy for 1-3 fractions. In some embodiments, the radiation is administered at a fraction dose of about 10-15 Gy for 1-3 fractions. In some embodiments, the radiation is administered at a fraction dose of about 15-20 Gy for 1-3 fractions. In some embodiments, the radiation is administered at a fraction dose of about 5-10 Gy for 1-3 fractions or 15-20 Gy for 1-3 fractions. In some embodiments, the radiation is administered at a fraction dose of about 8 Gy for 1 fraction. In some embodiments, the radiation is administered at a fraction dose of about 8 Gy for 3 fraction. In some embodiments, the radiation is administered at a fraction dose of about 16 Gy for 1 fraction. In some embodiments, the radiation is administered at a fraction dose of about 8 Gy for 1 fraction, or about 8 Gy for 3 fractions, or about 16 Gy for 1 fraction.

### Pharmaceutical Compositions

The STING agonists used in the methods and kits described herein can be formulated into pharmaceutical compositions suitable for administration.

The STING agonists and the checkpoint inhibitors used in the methods and kits described herein can be formulated into pharmaceutical compositions suitable for administration. The pharmaceutical compositions may comprise pharmaceutically acceptable excipients. A pharmaceutically acceptable excipient, as used herein, includes, but are not limited to, any and all solvents, dispersion media, or other liquid vehicles, dispersion or suspension aids, diluents, granulating and/or dispersing agents, surface active agents, isotonic agents, thickening or emulsifying agents, preservatives, binders, lubricants or oil, coloring, sweetening or flavoring agents, stabilizers, antioxidants, antimicrobial or antifungal agents, osmolality adjusting agents, pH adjusting agents, buffers, chelants, cyoprotectants, and/or bulking agents, as suited to the particular dosage form desired. Various excipients for formulating pharmaceutical compositions and techniques for preparing the composition are known in the art (*see* Remington: The Science and Practice of Pharmacy, 21st Ed., A. R. Gennaro (Lippincott, Williams & Wilkins, Baltimore, MD), 2006).

Any of the therapeutic agents described herein may be in the form of a pharmaceutically acceptable salt. In some embodiments, such salts are derived from inorganic or organic acids or bases. For reviews of suitable salts, *see, e.g.,* Berge et al., J. Pharm. Sci., 1977, 66, 1-19 and Remington: The Science and Practice of Pharmacy, 20th Ed., A. Gennaro (ed.), Lippincott Williams & Wilkins (2000).

Examples of suitable acid addition salts include acetate, adipate, alginate, aspartate, benzoate, benzene sulfonate, bisulfate, butyrate, citrate, camphorate, camphor sulfonate, cyclopentanepropionate, digluconate, dodecylsulfate, ethanesulfonate, fumarate, lucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate and undecanoate.

Examples of suitable base addition salts include ammonium salts; alkali metal salts, such as sodium and potassium salts; alkaline earth metal salts, such as calcium and magnesium salts; salts with organic bases, such as dicyclohexylamine salts, *N*-methyl-D-glucamine; and salts with amino acids such as arginine, lysine, and the like.

For example, Berge lists the following FDA-approved commercially marketed salts: anions acetate, besylate (benzenesulfonate), benzoate, bicarbonate, bitartrate, bromide, calcium edetate (ethylenediaminetetraacetate), camsylate (camphorsulfonate), carbonate, chloride, citrate, dihydrochloride, edetate (ethylenediaminetetraacetate), edisylate (1,2-ethanedisulfonate), estolate (lauryl sulfate), esylate (ethanesulfonate), fumarate, gluceptate (glucoheptonate), gluconate, glutamate, glycollylarsanilate (glycollamidophenylarsonate), hexylresorcinate, hydrabamine (*N,N'*-di(dehydroabietyl)-ethylenediamine), hydrobromide, hydrochloride, hydroxynaphthoate, iodide, isethionate (2-hydroxyethanesulfonate), lactate, lactobionate, malate, maleate, mandelate, mesylate (methanesulfonate), methylbromide, methylnitrate, methylsulfate, mucate, napsylate (2-naphthalenesulfonate), nitrate, pamoate (embonate), pantothenate, phosphate/diphosphate, polygalacturonate, salicylate, stearate, subacetate, succinate, sulfate, tannate, tartrate, teoclate (8-chlorotheophyllinate) and triethiodide; organic cations benzathine (*N,N'*-dibenzylethylenediamine), chloroprocaine, choline, diethanolamine, ethylenediamine, meglumine (*N*-methylglucamine) and procaine; and metallic cations aluminum, calcium, lithium, magnesium, potassium, sodium and zinc.

Berge additionally lists the following non-FDA-approved commercially marketed (outside the United States) salts: anions adipate, alginate, aminosalicylate, anhydromethylenecitrate, arecoline, aspartate, bisulfate, butylbromide, camphorate, digluconate, dihydrobromide, disuccinate, glycerophosphate, hemisulfate, hydrofluoride, hydroiodide, methylenebis(salicylate), napadisylate (1,5-naphthalenedisulfonate), oxalate, pectinate, persulfate, phenylethylbarbiturate, picrate, propionate, thiocyanate, tosylate and undecanoate; organic cations benethamine (*N*-benzylphenethylamine), clemizole (1 -*p*-chlorobenzyl-2-pyrrolildine-1'-ylmethylbenzimidazole), diethylamine, piperazine and tromethamine (tris(hydroxymethyl)aminomethane); and metallic cations barium and bismuth.

The pharmaceutical compositions may comprise pharmaceutically acceptable carriers. As used herein, "pharmaceutically acceptable carrier" refers to a material that is compatible with a recipient subject (a human) and is suitable for delivering an active agent to the target site without terminating the activity of the agent. The toxicity or adverse effects, if any, associated with the carrier preferably are commensurate with a reasonable risk/benefit ratio for the intended use of the active agent.

Pharmaceutically acceptable carriers that may be used in these compositions include ion exchangers, alumina, aluminum stearate, lecithin, serum proteins, such as human serum albumin, buffer substances such as phosphates or carbonates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The pharmaceutical compositions for use in the methods of the present disclosure may be manufactured by methods well known in the art such as conventional granulating, mixing, dissolving, encapsulating, lyophilizing, or emulsifying processes, among others. Compositions may be produced in various forms, including granules, precipitates, or particulates, powders, including freeze dried, rotary dried or spray dried powders, amorphous powders, tablets, capsules, syrup, suppositories, injections, emulsions, elixirs, suspensions or solutions. Formulations may contain stabilizers, pH modifiers, surfactants, solubilizing agents, bioavailability modifiers and combinations of these. These pharmaceutical compositions are formulated for pharmaceutical administration to a human being. Such compositions may be administered orally, parenterally, by inhalation spray, topically, rectally, nasally, buccally, vaginally or via an implanted reservoir. The term "parenteral" as used herein includes subcutaneous, intravenous, intraperitoneal, intramuscular, intra-articular, intra-synovial, intrasternal, intrathecal, intrahepatic, intralesional and intracranial injection or infusion techniques. In some embodiments, the compositions are administered orally, intravenously or subcutaneously. In some embodiments, the compositions are administered orally. In some embodiments, the compositions are administered intravenously. In some embodiments, the intravenous administration can be intravenous infusion or intravenous injection. In some embodiments, the compositions are administered by an intravenous infusion. In some embodiments, the compositions are administered by an intravenous injection. In some embodiments, the compositions are administered by subcutaneous injection. In some embodiments, the compositions are administered by intravenous infusion and then subsequently administered by subcutaneous injection. In another embodiment, the checkpoint inhibitor is coadministered with human hyaluronidase subcutaneously. These formulations may be designed to be short-acting, fast-releasing, or long-acting. Furthermore, the compositions may be administered in a local rather than systemic means, such as administration (*e.g*., by injection) at a tumor site.

Pharmaceutical formulations may be prepared as liquid suspensions or solutions using a liquid, such as an oil, water, an alcohol, and combinations of these. Solubilizing agents such as cyclodextrins may be included. Pharmaceutically suitable surfactants, suspending agents, or emulsifying agents, may be added for oral or parenteral administration. Suspensions may include oils, such as peanut oil, sesame oil, cottonseed oil, corn oil and olive oil. Suspension preparations may also contain esters of fatty acids such as ethyl oleate, isopropyl myristate, fatty acid glycerides and acetylated fatty acid glycerides. Suspension formulations may include alcohols, such as ethanol, isopropyl alcohol, hexadecyl alcohol, glycerol and propylene glycol; ethers, such as poly(ethyleneglycol); petroleum hydrocarbons such as mineral oil and petrolatum; and water.

Sterile injectable forms of these pharmaceutical compositions may be aqueous or oleaginous suspensions. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as sorbitan alkyl esters, such as Tweens or Spans, and other emulsifying agents or bioavailability enhancers which are commonly used in the manufacture of pharmaceutically acceptable solid, liquid, or other dosage forms may also be used for the purposes of formulation. Compounds may be formulated for parenteral administration by injection such as by bolus injection or continuous infusion. A unit dosage form for injection may be in ampoules or in multi-dose containers.

These pharmaceutical compositions may be orally administered in any orally acceptable dosage form including capsules, tablets, aqueous suspensions or solutions. When aqueous suspensions are required for oral use, the active ingredient is combined with emulsifying and suspending agents. If desired, certain sweetening, flavoring or coloring agents may also be added. For oral administration in a capsule form, useful diluents include lactose and dried cornstarch. In the case of tablets for oral use, carriers that are commonly used include lactose and corn starch. Lubricating agents, such as magnesium stearate, are also typically added. Coatings may be used for a variety of purposes, *e.g.,* to mask taste, to affect the site of dissolution or absorption, or to prolong drug action. Coatings may be applied to a tablet or to granulated particles for use in a capsule.

Alternatively, these pharmaceutical compositions may be administered in the form of suppositories for rectal administration. These may be prepared by mixing the agent with a suitable non-irritating excipient which is solid at room temperature but liquid at rectal temperature and therefore will melt in the rectum to release the drug. Such materials include cocoa butter, beeswax and polyethylene glycols.

These pharmaceutical compositions may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract may be effected in a rectal suppository formulation (see above) or in a suitable enema formulation. Topically-transdermal patches may also be used. For topical applications, the pharmaceutical compositions may be formulated in a suitable ointment containing the active component suspended or dissolved in one or more carriers. Carriers for topical administration of the compounds of the present disclosure include mineral oil, liquid petrolatum, white petrolatum, propylene glycol, polyoxyethylene, polyoxypropylene compound, emulsifying wax and water. Alternatively, the pharmaceutical compositions may be formulated in a suitable lotion or cream containing the active component(s) suspended or dissolved in one or more pharmaceutically acceptable carriers. Suitable carriers include mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions may be formulated in an ointment such as petrolatum.

The pharmaceutical compositions may also be administered by nasal aerosol or inhalation. Such compositions are prepared according to techniques well known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

In one embodiment, Compound No. 14 is formulated as a solution for intravenous infusion. In some embodiments, Compound No. 14 is formulated in a solution containing 3 mg/3 mL Compound No. 14 as free base. In one embodiment, the solution of Compound No. 14 can be diluted prior to infusion.

### Kits

In some embodiments, the STING agonist or the checkpoint inhibitor described herein may be manufactured for inclusion in a kit. A "kit" is any article of manufacture (*e.g.,* a package or container) comprising at least one reagent or chemotherapeutic agent. A kit for use in the methods herein may comprise a STING agonist, such as Compound No. 14 or a pharmaceutically acceptable salt thereof. In some embodiments, the kit may further include a checkpoint inhibitor, and optionally one or more additional therapeutic agents. In some embodiments, the kit may include Compound No. 14 or a pharmaceutically acceptable salt thereof, a checkpoint inhibitor, and optionally one or more additional therapeutic agents. In some embodiments, the kit may include one or more STING agonists or pharmaceutically acceptable salts thereof. In some embodiments, the kit may include one or more checkpoint inhibitors. In some embodiments, the kay may further include instructions for administering radiation.

In some embodiments, the present disclosure relates to a kit comprising a medicament for use in treating cancer in a patient in need of such treatment. The kit comprises a medicament comprising a STING agonist, instructions for administering the STING agonist and a checkpoint inhibitor, and instructions for administering radiation; or the kit may comprise a medicament comprising a checkpoint inhibitor, instructions for administering the checkpoint inhibitor and a STING agonist, and instructions for administering radiation. The kit may contain a medicament comprising a STING agonist and a checkpoint inhibitor, instructions for administering the STING agonist and the checkpoint inhibitor, and instructions for administering radiation, wherein the medicament is in single dosage form or in separate dosage forms. In some embodiments, the kit optionally comprises one or more additional therapeutic agents.

In some embodiments, a kit comprising a STING agonist, a checkpoint inhibitor, and instructions for administering radiation, may further include another component or reagent. In some embodiments, a reagent in the kit may be a diluent for preparing the STING agonist for administration. In some embodiments, a reagent in the kit may be a diluent for preparing the checkpoint inhibitor for administration. In some embodiments, a component in the kit may be a vessel for mixing the combination of the STING agonist and the checkpoint inhibitor.

In another aspect, the present disclosure relates to a kit for treating cancer comprising at least one medicament comprising at least one dose of Compound No. 14 or a pharmaceutically acceptable salt thereof, at least one medicament comprising at least one dose of a checkpoint inhibitor, and instructions for administering radiation, said kit for treating cancer further comprising dosing instructions for administering the medicaments and radiation for treatment of the patient in recognized need thereof.

In order that this present disclosure be more fully understood, the following examples are set forth. These examples are illustrative only and are not intended to limit the scope of the present disclosure in any way.

### EXAMPLES

### Abbreviations

- H: hour
- Min: minutes
- HPLC: High-pressure liquid chromatography
- UPLC: Ultra-pressure liquid chromatography
- NMR: Nuclear Magnetic Resonance
- THF: tetrahydrofuran
- WFI: Water for Injection
- TGI: tumor growth inhibition
- Mg: milligram
- kg: kilogram
- mm³: cubic millimeter
- HPbCD: 2-hydroxypropyl-β-cyclodextrin
- CMC: carboxymethylcellulose
- PO: oral
- AE: adverse event
- SC: Subcutaneously
- Gy: grays
- mL: milliliters
- mpk: milligrams per kilogram
- PBS: phosphate buffered saline
- GRI: growth rate inhibition
- IP: intraperitoneal
- SD: starting day
- SA: single agent
- Q3W: once every 3 weeks
- Q3D: every third day
- BLRM: Bayesian Logistic Regression Modeling
- MTD: maximum tolerated dose
- PAD: pharmacologically active dose
- IV: intravenous
- DLT: Dose limiting toxicity
- PK: Pharmacokinetic
- TEAEs: Treatment-emergent adverse events
- TESAE: Treatment-emergent serious adverse events
- DL: Dose level
- RP2D: recommended phase 2 dose
- MABEL: minimum anticipated biological effect level
- SOE: schedule of events
- CR: complete responders
- cPR: confirmed partial response
- NCI CTCAE: National Cancer Institute Common Terminology Criteria for Adverse Events

### Example 1: In Vivo Tumor Efficacy

### General Experimental Conditions for Anti-Tumor Efficacy in Mouse Tumor Models

### Mouse Syngeneic Tumor Models

The following syngeneic models were utilized in each of Studies 1-3, as specified below.

Studies 1 and 2: EMT6 is a mouse breast carcinoma cell line. EMT6 mouse syngeneic tumor model was generated by subcutaneous (sc) inoculation with 0.2 x 10⁶ EMT6 cells (cell suspension) in nine to ten week old female BALB/c mice (Vital River Laboratory Animal Technology Co., Ltd., Beijing, China) in the flank. When the mean tumor volume reached 60 mm³ in Study 1 and 75 mm³ in Study 2 the animals were randomized into one vehicle control and seven treatment groups (n=10/group). Mice were then dosed with vehicle (phosphate buffered saline (PBS)) or Compound No.14 or 10 Gy Radiation or Compound No.14 plus 10Gy Radiation over an eight-day period. Tumor growth and body weight were measured three times per week during the treatment and post-treatment periods. Mice were humanely euthanized once they reached their humane endpoint or until study termination on day 72 (Study 1) or on day 60 (Study 2).

Study 3: EMT6 is a mouse breast carcinoma cell line. EMT6 mouse syngeneic tumor model was generated by orthotopic inoculation with 1.0 x 10⁶ EMT6 cells (cell suspension) in seven to eight week old female BALB/c mice (Envigo Corp., Indianapolis, IN) in the mammary fat pad. When the mean tumor volume reached approximately 110 mm³, the animals were randomized into one vehicle control and eight treatment groups (n=8/group). Mice were then treated with Compound No. 14, anti-mouse PD-1 (anti-mPD-1) (clone RMP1-14), and/or three fraction doses of 8Gy of Radiation (8 Gy x 3), or the respective control agents (PBS, isotype control antibody, and/or mock radiation) in the following combinations over an 11-day period.
Group 1: vehicle (PBS) + isotype control + mock radiation
Group 2: Compound No. 14 (1 mg/kg) + isotype control + mock radiation
Group 3: Compound No. 14 (0.25 mg/kg) + isotype control + mock radiation
Group 4: vehicle + anti-mPD-1 + mock radiation
Group 5: vehicle + isotype control + radiation (8 Gy x 3)
Group 6: Compound No. 14 (1 mg/kg) + isotype control + radiation (8 Gy x 3)
Group 7: vehicle + anti-mPD-1 + radiation (8 Gy x 3)
Group 8: Compound No. 14 (1 mg/kg) + anti-mPD-1 + radiation (8 Gy x 3)
Group 9: Compound No. 14 (0.25 mg/kg) + anti-mPD-1 + radiation (8 Gy x 3)

Tumor growth and body weight were measured three times per week during the treatment and post-treatment periods. Mice were humanely euthanized once they had reached their humane endpoint. Mice with complete regressions were evaluated until day 74 or day 108.

Study 4: CT26 is a murine colorectal carcinoma cell line. Eight-week-old female Balb/c mice (Jackson Labs, Maine) were inoculated subcutaneously with 0.2x10⁶ CT26 tumor cells and monitored for tumor growth. Once the tumors reached an average of 100-125 mm³, animals were irradiated with either mock irradiation or 8 Gy of focal beam irradiation (Study Day 0). Animals were then dosed intravenously with either vehicle (PBS) or 0.25 mg/kg of Compound No.14 on a QWx3 schedule beginning on Study Day 1. Anti-mPD-1 antibody (clone RMP1-14, BioXCell) treatment was administered intraperitoneally twice weekly starting on Study Day 1 with a concentration of 10 mg/kg for 3 weeks. Animals were monitored for tumor volumes and body weight changes twice weekly throughout the study once they reached their humane endpoint. GRI was calculated on the last day with animals remaining in the vehicle group (Study Day 11).

Study 5: B16F10-Ova is a cell line derived from B16F10 melanoma that expresses chicken ovalbumin. Seven-week-old female C57Bl/6 mice (Jackson Labs, Maine) were inoculated subcutaneously with 0.04 x10⁶ B16F10 ova tumor cells and monitored for tumor growth. Once the tumors reached an average of 100-250 mm³, animals were irradiated with either mock irradiation or 16 Gy of focal beam irradiation (Study Day 0). Animals were then dosed intravenously with either vehicle (PBS) or 0.25 mg/kg of Compound No.14 on a Q3Dx3 scheduled beginning on Study Day 1. Animals were monitored for tumor volumes and body weight changes twice weekly throughout the study once they reached their humane endpoint. GRI was calculated on the last day with animals remaining in the vehicle group (Study Day 10).

### Test Agents

The following test agents were utilized in Studies 1-3, as specified below.

Studies 1 and 2: A 0.1 mg/mL stock solution of Compound No.14 was formulated in PBS and administered intravenously (IV) based on exact animal body weight on each day of treatment, using a dosing volume of 10 mL/kg body weight. Final doses received were 1.0 mg/kg. Dosing volume for Compound No.14 did not exceed 0.2 mL. Compound No.14 was administered on a Q3D schedule for 3 doses (Day 2, 5 and 8) in single agent treatment group and in combination treatment groups. Dose formulation of Compound No. 14 was prepared once and utilized for dosing on Days 2, 5 and 8.

Radiation treatment for Studies 1 and 2 was performed using RadSource RS 2000 X-ray irradiator (Rad Source Technologies). Animals received 1.046 Gy/minute of radiation and approximately 9.5 min was needed to reach a 10Gy dose delivery. Animals were anesthetized prior to placement in irradiation chamber using isoflurane and checked for reflexes. Radiation beam was focused on tumor with manual positioning. Radiation was administered via manual positioning in a single 10Gy dose on either day 0, 2 or 4 in Study 1, and on either day 0, 1, 2, 3 or 4 in Study 2.

Study 3: A 0.1 mg/mL stock solution of Compound No.14 was formulated in PBS and administered IV based on exact animal body weight on each day of treatment, using a dosing volume of 10 mL/kg body weight. Final doses received were 0.25mg/kg or 1.0 mg/kg. Compound No. 14 was administered on a Q3D schedule for 3 doses (Days 12, 15 and 18) in single agent treatment group and in combination treatment groups. Dose formulation of Compound No. 14 was prepared once and utilized for all three dosing days.

Anti-mPD-1 antibody (clone RMP1-14) and isotype control which does not bind to mPD-1 (mIgG2a, clone 2A3) (both from Bio X Cell, 10 Technology Drive, Suite 2B, West Lebanon, NH 03784) were formulated prior to the first injection at 1.0 mg/mL in PBS on Day 12 and administered intraperitoneally (IP) based on exact body weight on each day of treatment, using a dosing volume of 0.01 mL/g resulting in a 10 mg/kg dose. Formulated anti-mPD-1 and isotype control were stored at 4 degree Celsius until subsequent dosing on Days 15 and 18. Anti-mPD-1 or isotype control was administered concurrent with Compound No.14 or vehicle control on a Q3D schedule for 3 doses (Days 12, 15 and 18) in single agent treatment groups and in combination treatment groups.

Radiation treatment for Study 3 was performed using focal beam radiation with volumetric image guidance from computed tomography scans SARRP (Small Animal Radiation Research Platform) irradiator (Xstrahl Inc. Suwanee, Ga). Animals received 2.42 Gy/min of radiation and approximately 3.3 min was needed to reach 8Gy dose delivery. Animals received 8Gy radiation on days 8, 9 and 10, with the last radiation dose given two days prior to the initiation of dosing with Compound No.14 and/or anti-mPD-1 or relevant controls on Day 12. Animals were anesthetized during imaging and radiation treatment using 1-2% Isoflurane. For repeated daily radiation treatments, the same treatment plan was applied and adjusted for changes in animal positioning or target alteration over time. Animals receiving mock radiation did not receive any radiation but merely were anesthetized for an equal time as those receiving radiation to control for the non-radiation aspects of the procedure.

Study 4: A 5 mg/mL stock solution of Compound No.14 was formulated in PBS at the final concentration of 0.0667 mg/mL and administered intravenously (IV). Final doses received were 0.25 mg/kg. Dosing volume for Compound No. 14 did not exceed 0.2 mL. Compound No. 14 was administered on a QW schedule for 3 doses in single agent treatment group and in combination treatment groups beginning on Day 1. Dose formulation of Compound No.14 was prepared once and utilized for dosing on each treatment day.

Study 5: A 5 mg/mL stock solution of Compound No.14 was formulated in PBS at the final concentration of 0.0667 mg/mL and administered intravenously (IV). Final doses received were 0.25 mg/kg. Dosing volume for Compound No. 14 did not exceed 0.2 mL. Compound No. 14 was administered on a Q3D schedule for 3 doses in single agent treatment group and in combination treatment groups beginning on Day 1. Dose formulation of Compound No.14 was prepared once and utilized for dosing on each treatment day.

Studies 4 and 5: Anti-mPD-1 antibody (clone RMP1-14) and isotype control which does not bind to mPD-1 (mIgG2a, clone 2A3) (both from Bio X Cell, 10 Technology Drive, Suite 2B, West Lebanon, NH 03784) were formulated prior to each injection at 2.1 mg/mL in PBS and administered intraperitoneally (IP), using a dosing volume of 0.1 mL/mouse resulting in a 10 mg/kg dose. Anti-mPD-1 or isotype control was administered concurrent with Compound No. 14 or vehicle control on a BIW schedule for 3 doses in single agent treatment groups and in combination treatment groups.

Studies 4 and 5: Radiation treatment for Studies 4 and 5 were performed using focal beam radiation with volumentric image guidance from computer tomography scans on the SmART (Small animal image-guided radiotherapy) platform (Precision XRay Inc., North Branford, CT). Animals received 4.52 Gy/min of radiation and approximately 1.8 min was needed to reach 8 Gy dose delivery (Study 4) or 2.6 min was needed to reach 16 Gy dose delivery (Study 5). Animals were anesthetized during imaging and radiation treatment using 1-2% isoflurane.

### Tumor Measurements

Tumors were measured three times weekly using vernier calipers. Tumor volumes were calculated using standard equation: V = W² x L/2, where V = volume, W = width, and L = length for the tumor. When mean tumor volumes reached approximately 60 mm³ for Study 1, 75 mm³ for Study 2, and 110 mm³ for Study 3, mice were randomized into eight groups (n=10/group) in Studies 1 and 2, and nine groups (n=8/group) in Study 3. These groups were then dosed with vehicle (PBS), Compound No.14, Radiation, anti-mPD-1 or isotype control, or combinations of these agents as described in Tables 1a, 2a, and 3a. Tumor size and body weight were measured three times a week for the duration of each study. Mice were euthanized for Studies 1 and 2 when their tumor volumes reached greater than 10% of their body weight or when an individual tumor exceeded the humane end-point for size (>2 cm) or when an animal lost 20% of their body weight relative to the start of the study or lost 15% of their body weight in any 24 hr time frame. Mice were euthanized in Study 3 when tumor volume exceeded 2,000 mm³ or an animal lost more than 20% of their body weight relative to the start of study.

### Statistical Analysis of Comparing Different Treatments in Mouse Syngeneic Tumor Models

The following statistical analysis methods were utilized in Studies 1, 2, 4 and 5.

**Growth Rate Inhibition:** The differences in the tumor growth trends over time between pairs of treatment groups were assessed by fitting each animal's data to a simple exponential growth model and comparing the mean growth rates of the two groups, as described in more detail below. The difference in the growth rates was summarized by the GRI, which is the reduction in growth rate experienced by the treatment group relative to that of the reference group, expressed as a fraction of the vehicle growth rate. $Growth rate inhibition = \frac{{μ}_{C} - {μ}_{T}}{{μ}_{V}} \times 100 %$

µ*_{T}* and µ*_{C}* are the mean tumor growth rates for the treatment and reference groups, respectively. µ*_{V}* is the mean tumor growth rate for the vehicle group, which in most cases is the same as the reference group.

A positive GRI indicates that the tumors in the treatment group grew at a reduced rate relative to the reference group. A GRI greater than 100% is interpreted as tumor regression when the vehicle group is the same as the reference group. A negative value of the GRI means that the tumors in the treatment group grew faster than the tumors in the reference group. Statistical significance was determined using an unpaired t-test. A statistically significant P value suggests that the trends over time for the two treatment groups were different.

**Tumor Growth Rate Computation:** Low tumor volumes can cause problems with the data analysis, so all measurements below 25 cubic mm (including 0 values) were excluded from the analysis. After this exclusion step, the tumor volume was assumed to follow an exponential growth model. More specifically, for a given animal and treatment group, ${log}_{10} \left({V}_{i}\right) = a + b \times {t}_{i} + {ε}_{i}$ where *Vᵢ* is the tumor volume at the *i^{th}* time point. Here, *a* is the initial log volume, *b* is the tumor growth rate, and *tᵢ* is the measurement time in days. *εᵢ* is the residual error term, which was assumed to be uncorrelated and drawn from a normal distribution.

This model was fit separately for each animal within each treatment group. If an animal was sacrificed or died early, but at least two unique time points were measured (including baseline), then the data up to that point was used to estimate the growth rate. If the animal had measurements at less than two time points, then the animal was automatically excluded from the analysis.

In rare cases, the estimated growth rate for one or more animals might be very different from the other animals within the same group. To make the analysis robust, an interval was defined with a width of 30 times the median absolute deviation of the estimated growth rates for a given group. The interval was centered at the median of the growth rates for the group. If the growth rate for any animal fell outside this interval, the growth rate was replaced with the value at the boundary of the interval.

**Combination Analysis:** Combination analysis was performed to determine if there was a benefit from combining drug treatments. This analysis was also based on the estimated tumor growth rates. The measure of synergy was defined as $Synergy score = \frac{{μ}_{AB} - {μ}_{A} - {μ}_{B} + {μ}_{Control}}{{μ}_{V}} \times 100 %$

Here, *µ_{AB}, µ_{A}, µ_{B},* and *µ_{Control}* are the mean growth rates for the combination, drug A, drug B, and control groups, respectively. As before, *µ_{V}* is the mean tumor growth rate for the vehicle group, which in most cases is the same as the control group. The standard error of the synergy score was calculated as the square root of the sum of squared standard errors across the four groups. The degrees of freedom were estimated using the Welch-Satterthwaite equation. A hypothesis test was performed to determine if the synergy score differed from 0. P-values were calculated by dividing the synergy score by its standard error and tested against a t-distribution (two-tailed) with the above-calculated degrees of freedom. A P value of less than 0.05 is statistically significant.

The combination results can be interpreted as follows. Statistically significant negative synergy scores indicate a synergistic combination. Statistically significant positive synergy scores indicate a sub-additive combination when the combination performs better (i.e. has a lower growth rate) than the best performing single agent. Statistically significant positive synergy scores indicate an antagonistic combination when the combination performs worse than the best performing single agent. Scores that are not statistically significant should be considered additive.

The following statistical analysis methods were utilized in Study 3.

**Pairwise comparisons to vehicle group:** All tumor volumes had a value of 5 added to them before log transformation. After the transformation, linear interpolation was used to estimate the time (in days) since randomization when each mouse's tumor volume reaches 1000 mm³. We consider the event of a mouse's tumor reaching 1000 mm³ as "tumor progression" event and call the aforementioned estimated time as time-to-progression (TTP). In case of a mouse's tumor having not reached 1000 mm³ at the end of study, the last day the mouse was on study was recorded and right-censored. For each pair of treatments of interest, a parametric survival model that assumes a Weibull distribution on TTP was used to estimate the hazard ratio (HR) between two treatment arms. HR reflects the ratio of the hazards the mice of the two treatment arms experience progression events at any timepoint throughout the study. A HR between treatment A and B that is smaller than 1 suggests better efficacy for treatment A than B. Standard errors (SE) and 95% confidence intervals (CI) were also calculated to describe the uncertainties of the estimated HRs. Finally, two types of tests were used to calculate the P-values in order to assess the statistical significance of the difference between the two treatments: 1) A Wald test p-value from the Weibull survival model; 2) a non-parametric log-rank test.

**Combination Treatment Effects:** Combination benefit was also based on TTP. The synergy is assessed from the Weibull model. Synergy HR (Hazard Ratio) is interpreted as ${λ}_{synergy} = \frac{{λ}_{AB \left|Ctrl\right.}}{{λ}_{A \left|Ctrl\right.} ∗ {λ}_{B \left|Ctrl\right.}}$ Where, *λ*_{*A*|*Ctrl*} and *λ_{B}*|*_{Ctrl}* are, respectively, the hazard ratios for comparing the treatments A and B to vehicle control group, and λ*_{AB}*|*_{cCtrl}* is the hazard ratio comparing the combination to the vehicle group. If the estimate of *λ_{synergy}* is significantly less than 1, that means that a synergy exists between the two treatment arms. In practice, instead of calculating the *λ_{synergy}* using the three individually estimated hazard ratios, a Weibull regression model is fitted to all 4 treatment groups with terms for treatments A and B, and their interaction. The interaction term can hence be interpreted as l*o*g(*λ_{synergy}*). Thus, the *λ_{synergy}* is calculated by exponentiating the estimated interaction term, whose p-value is obtained by testing whether the interaction term is significantly different from zero on log-scale.

### Results

### Study 1:

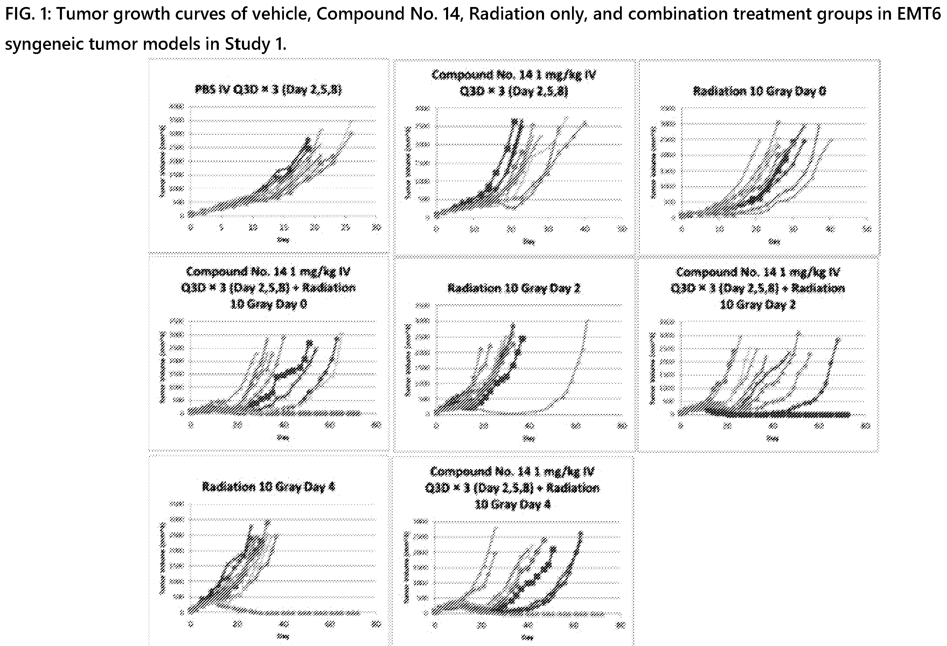

The tumor growth rate inhibition of the treatment groups from Study 1 is shown in Table 1a. The combination effect for the period through Day 19 is shown in Table 1b. Tumor growth curves for each group are shown in Figure 1.

All treatment groups showed statistically significant GRI on Day 19 compared to the vehicle group. Synergy analysis conducted using data through Day 19 showed additive benefit of Compound No.14 plus Radiation (10Gy on Days 0, 2 or 4) on all 3 schedules when compared to single agent Compound No.14 and the matched Radiation-only groups. Although synergistic benefits were not detected during this 19-day period, during the remaining portion of the 72-day study, more complete regressions were achieved in the combination treatment groups compared to the Radiation-only groups or the single agent Compound No. 14 group. There were 1, 1, and 2 complete responders (CRs) detected when Compound No. 14 was administered in combination with Radiation on Days 0, 2, and 4, respectively. In contrast, there were 0, 0, and 1 CRs in the Radiation-only groups (which received Radiation on Days 0, 2, or 4 respectively), and 0 CRs in the single agent Compound No. 14 group.

**Table 1a: Study 1 Tumor Growth Rate Inhibition and Complete Regressions**

| **Model** | **Study Number** | **Treatment Group** | **GRI (%) Day 19** | **P value** | **Number of Complete Regressions** |
|---|---|---|---|---|---|
| EMT6 | 1 | Compound No. 14 | 30 | <0.001 | 0 |
| EMT6 | 1 | 10Gy Radiation (Day 0) | 32 | 0.009 | 0 |
| EMT6 | 1 | Compound No. 14 + 10Gy Radiation (Day 0) | 82 | <0.001 | 1 |
| EMT6 | 1 | 10Gy Radiation (Day 2) | 36 | 0.001 | 0 |
| EMT6 | 1 | Compound No. 14 + 10Gy Radiation (Day 2) | 60 | 0.001 | 1 |
| EMT6 | 1 | 10Gy Radiation (Day 4) | 27 | 0.007 | 1 |
| EMT6 | 1 | Compound No. 14 + 10Gy Radiation (Day 4) | 67 | <0.001 | 2 |

**Table 1b: Study 1 Combination Assessment**

| **Model** | **Study Number** | **Combination Group** | **Synergy Score %** | **P Value** | **Combination Assessment** |
|---|---|---|---|---|---|
| EMT6 | 1 | Compound No.14 + Radiation (10 Gy) Day 0 | -20 | 0.176 | Additive |
| EMT6 | 1 | Compound No.14 + Radiation (10 Gy) Day 2 | 7 | 0.654 | Additive |
| EMT6 | 1 | Compound No.14 + Radiation (10 Gy) Day 4 | -10 | 0.487 | Additive |

### Study 2:

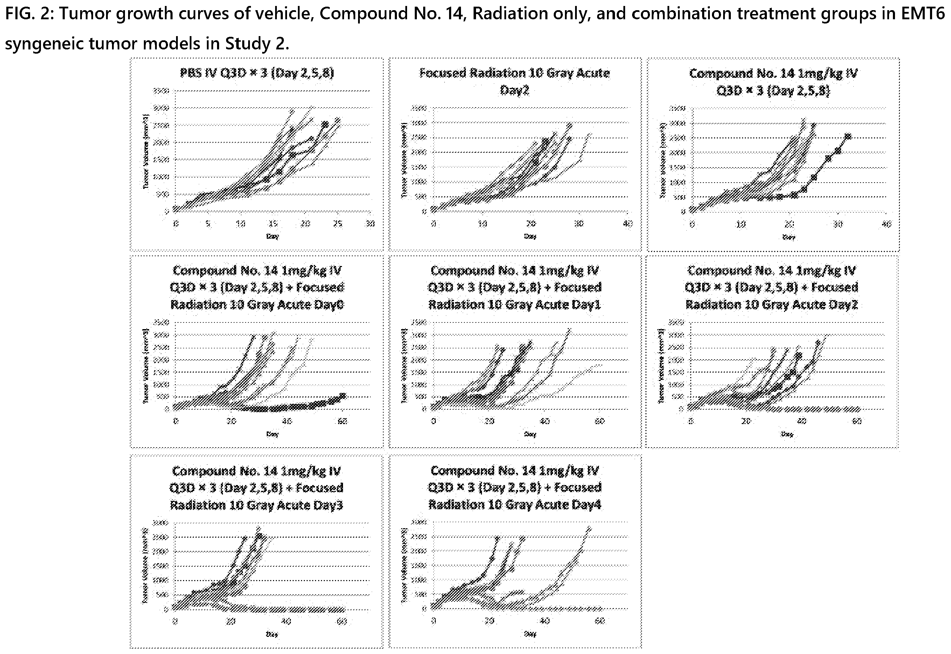

The tumor growth rate inhibition of treatment groups from Study 2 is shown in Table 2a. The combination effect for the period through Day 21 is shown in Table 2b. Tumor growth curves for each group are shown in Figure 2.

All treatment groups showed statistically significant GRI on Day 21 compared to the vehicle group. Combination analysis conducted using data through study Day 21 indicated that the combination of Compound No. 14 plus Radiation (10 Gy on Day 2) was synergistic when compared to single agent Compound No. 14 and Radiation-only (10 Gy on Day 2) groups. The combination of Compound No.14 plus Radiation on other schedules (10Gy on Day 0, 1, 3, or 4) yielded additive benefits when compared to single agent Compound No.14 and Radiation-only arms. During the remaining portion of the 60-day study, more CRs were achieved in three of the combination treatment groups compared to the Radiation-only group or the single agent Compound No. 14 group. There were 2, 4, and 2 CRs detected when Compound No. 14 was administered in combination with Radiation on Days 2, 3, and 4, respectively (Table 2a). In contrast, there were no CRs in the Radiation-only group, the single agent Compound No. 14 group or the combination group in which Radiation was administered on Day 0 or 1.

**Table 2a: Study 2 Tumor Growth Rate Inhibition and Complete Regressions**

| **Model** | **Study Number** | **Treatment Group** | **GRI (%) Day 21** | **P value** | **Complete Regressions** |
|---|---|---|---|---|---|
| EMT6 | 2 | Radiation (10Gy) (Day 2) | 20 | 0.003 | 0 |
| EMT6 | 2 | Compound No. 14 | 20 | 0.011 | 0 |
| EMT6 | 2 | Compound No. 14 + Radiation (10Gy) (Day 0) | 60 | <0.001 | 0 |
| EMT6 | 2 | Compound No. 14 + Radiation (10Gy) (Day 1) | 62 | 0.001 | 0 |
| EMT6 | 2 | Compound No. 14 + Radiation (10Gy) (Day 2) | 68 | <0.001 | 2 |
| EMT6 | 2 | Compound No. 14 + Radiation (10Gy) (Day 3) | 64 | <0.001 | 4 |
| EMT6 | 2 | Compound No. 14 + Radiation (10Gy) (Day 4) | 60 | <0.001 | 2 |

**Table 2b: Study 2 Combination Assessment**

| **Model** | Study Number | Combination group | Synergy Score % | P Value | Combination Assessment |
|---|---|---|---|---|---|
| EMT6 | 2 | Compound No.14 + Radiation (10Gy) (Day 0) | -20 | 0.124 | Additive |
| EMT6 | 2 | Compound No.14 + Radiation (10Gy) (Day 1) | -22 | 0.151 | Additive |
| EMT6 | 2 | Compound No.14 + Radiation (10Gy) (Day2) | -28 | 0.04 | Synergistic |
| EMT6 | 2 | Compound No.14 + Radiation (10Gy) (Day 3) | -24 | 0.085 | Additive |
| EMT6 | 2 | Compound No.14 + Radiation (10Gy) (Day 4) | -20 | 0.124 | Additive |

### Study 3:

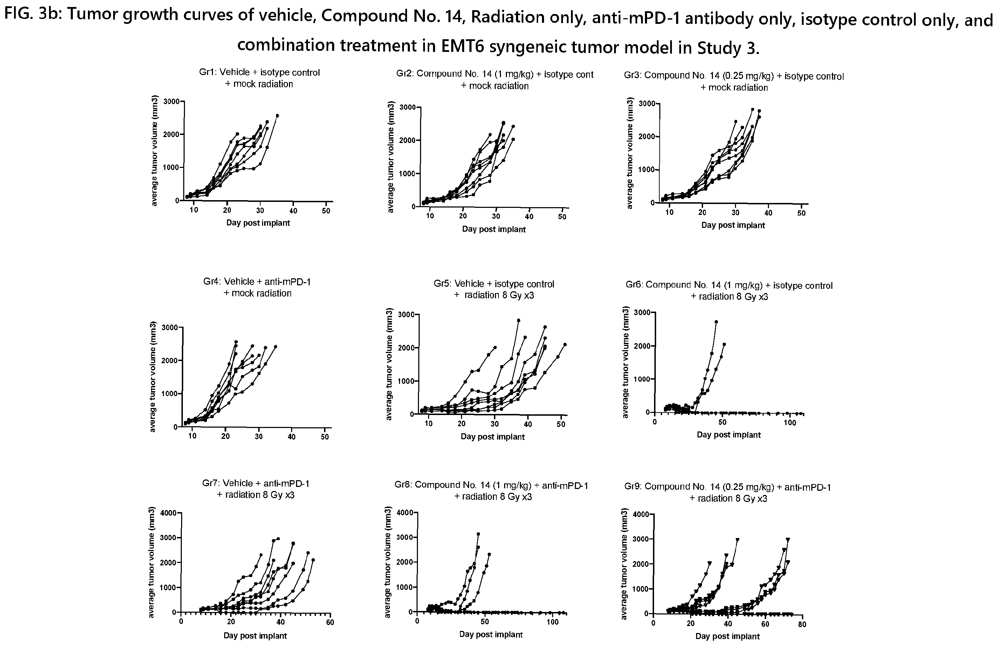

The time-to-progression analysis of treatment groups from Study 3 is shown in Table 3a. The combination analysis results are shown in Table 3b. Kaplan-Meier survival curves for each group are shown in Figure 3a. Tumor growth curves are shown in Figure 3b.

All treatment groups which included Compound No. 14 (0.25 mg/kg or 1 mg/kg) or 8 Gy x 3 Radiation demonstrated a statistically significant increase in TTP (time to tumor volume reaching 1000 mm³) compared to the control group. Anti-mPD1 as a single agent did not significantly increase TTP compared to control. Statistically significant synergy was demonstrated between Compound No. 14 (1 mg/kg) and 8 Gy x 3 Radiation with anti-mPD1 (Group 8) or with isotype control (Group 6). Significant synergistic activity was also demonstrated between Compound No. 14 (0.25 mg/kg) and 8 Gy x 3 Radiation and anti-mPD1 (Group 9). In contrast, the combination of anti-mPD 1 and 8 Gy x 3 Radiation was not synergistic in this experiment (Group 7).

Survival curves for the study in Figure 3a represent the number of mice remaining on study in each group on a given study day. Mice were removed from study when tumor volume exceeded 2,000 mm³ or an animal lost more than 20% of their body weight relative to the start of study. The survival graph shows that 5 mice in Group 6 [Compound No. 14 (1 mg/kg) + isotype control + 8 Gy x 3 Radiation] and 5 mice in Group 8 [Compound No. 14 (1 mg/kg) + anti-mPD1 + 8 Gy x 3 Radiation] remained on study as tumor-free survivors at end of study (Day 108). In contrast, no mice in the relevant comparison groups (Gr 2, 5, 7) remained on study beyond day 53, indicating a durable benefit of these combinations.

**Table 3a: Study 3 Time to Progression analysis**

| **Model** | **Study No.** | **Pairwise comparison to vehicle group (Gr 1)** | **HR (Weibull)** | **95% CI (Weibull)** | **SE (Weibull)** | **P-value (Weibull)** | **P-value (log-rank)** |
|---|---|---|---|---|---|---|---|
| EMT6 | 3 | Gr 2 | 0.485 | (0.18, 1.3) | 0.245 | 0.151 | 0.0312* |
| EMT6 | 3 | Gr 3 | 0.37 | (0.135, 1.02) | 0.191 | 0.048 | 0.0251 * |
| EMT6 | 3 | Gr 4 | 2.74 | (0.898, 8.38) | 1.56 | 0.0473 | 0.309 |
| EMT6 | 3 | Gr 5 | 0.0313 | (0.0069, 0.142) | 0.0242 | 6.2e-12 | 0.000306* |
| EMT6 | 3 | Gr 6 | 0.00091 | (2.93e-05, 0.0283) | 0.0016 | 3.72e-16 | 3.85e-05* |
| EMT6 | 3 | Gr 7 | 0.0395 | (0.00827, 0.189) | 0.0315 | 1.06e-10 | 9.91e-05* |
| EMT6 | 3 | Gr 8 | 0.00376 | (0.000211, 0.0671) | 0.00553 | 2.44e-15 | 3.85e-05* |
| EMT6 | 3 | Gr 9 | 0.0174 | (0.00234, 0.129) | 0.0178 | 4.86e-15 | 3.69e-05* |

| | | | | | | | |
|---|---|---|---|---|---|---|---|
| *Statistically significant Hazard Ratio based upon log-rank test. | | | | | | | |

**Table 3b: Study 3 Combination Assessment**

| Model | Study No. | Combination Group | Comparison Group A | Comparison Group B | Synergy Hazard Ratio (Weibull) | CI_95% (Weibull) | S.E. (Weibull) | P-value (Weibull) |
|---|---|---|---|---|---|---|---|---|
| EMT6 | 3 | Gr 6 [Compound No.14 (1 mg/kg) + isotype control + radiation (8 Gy x 3)] | Gr 2 [Compound No. 14 (1 mg/kg) + isotype control + mock radiation] | Gr 5 [vehicle + isotype control + radiation (8 Gy x 3)] | 0.00194 | (0.000166, 0.0226) | 0.00243 | 4.8e-11* |
| EMT6 | 3 | Gr 9 [Compound No.14 (0.25 mg/kg) + anti-mPD-1 + radiation (8 Gy x 3)] | Gr 3 [Compound No. 14 (0.25 mg/kg) + isotype control + mock radiation] | Gr 7 [vehicle + Anti-mPD-1 + radiation (8 Gy x 3)] | 0.214 | (0.0488, 0.938) | 0.161 | 0.0322* |
| EMT6 | 3 | Gr 8 [Compound No.14 (1 mg/kg) + anti-mPD-1 + radiation (8 Gy x 3)] | Gr 2 [Compound No. 14 (1 mg/kg) + isotype control + mock radiation] | Gr 7 [vehicle + Anti-mPD-1 + radiation (8 Gy x 3)] | 0.00993 | (0.0013, 0.0758) | 0.0103 | 6.15e-08* |
| EMT6 | 3 | Gr 7 [vehicle + anti-mPD-1 + radiation (8 Gy x 3)] | Gr 4 [vehicle + anti-mPD 1+ mock radiation] | Gr 5 [vehicle + isotype control + radiation (8 Gy x 3)] | 0.272 | (0.061, 1.21) | 0.207 | 0.0686 |

| | | | | | | | | |
|---|---|---|---|---|---|---|---|---|
| *Statistically significant Synergy Hazard Ratio | | | | | | | | |

### Study 4:

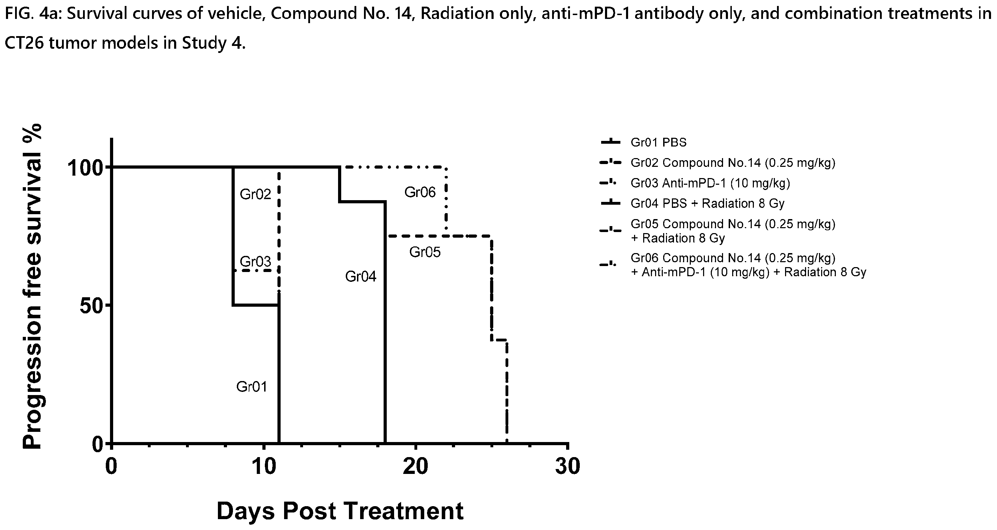

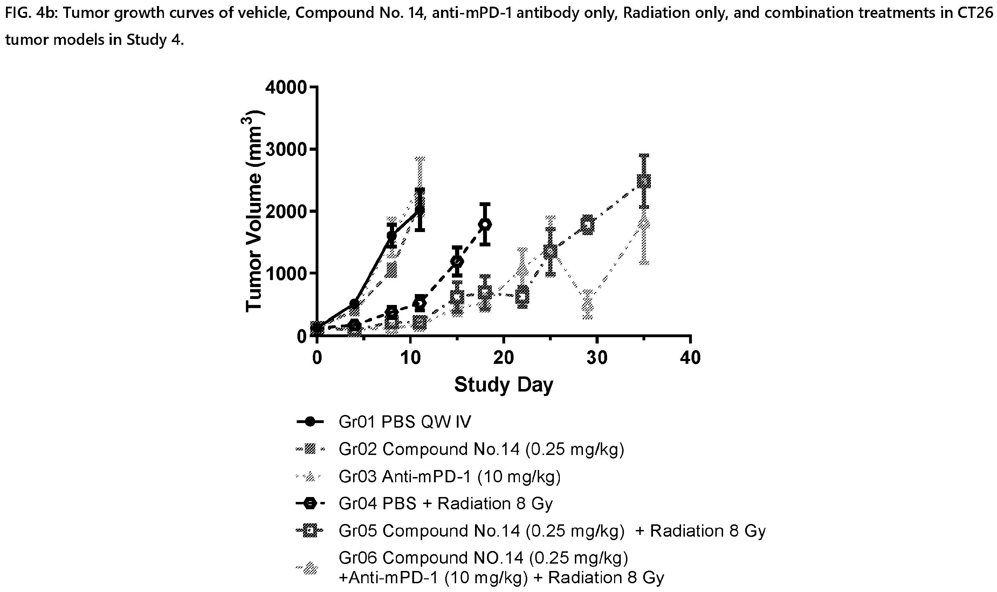

The tumor growth rate inhibition of treatment groups from Study 4 is shown in Table 4a. The combination effect for the period through Day 11 is shown in Table 4b. Kaplan-Meier survival curves are shown in Figure 4a, and tumor growth curves for each group are shown in Figure 4b.

Anti-tumor activity (when compared with vehicle) was the greatest in animals that were treated with the triple combination of Compound No. 14, plus radiation, plus anti-mPD-1 treatment (GRI of 101%, p value <0.001). The combination of Compound No.14 with radiation also demonstrated significant anti-tumor activity when compared to vehicle, although to a lesser extent than the triple combination (GRI of 88%, p value < 0.001), and radiation treatment alone also demonstrated moderate antitumor activity when compared to vehicle, although this was less robust than either the double or triple combination groups (63%, p value = 0.001). In contrast, insignificant anti-tumor activity was observed in animals treated with either Compound No. 14 or anti-PD-1 treatments alone (GRI 13%, p value 0.698, or GRI -2%, p value = 0.792, respectively). These treatments were all well tolerated with no animals removed from study for body weight loss greater than 20 percent.

**Table 4a: Study 4 Tumor Growth Rate Inhibition and Complete Regressions**

| **Model** | **Study Number** | **Treatment Group** | **GRI (%) Day 11** | **P value** | **Number of Animals Removed** |
|---|---|---|---|---|---|
| CT26 | 4 | TG01 Vehicle QW IV (reference group) | N/A | N/A | 4 |
| CT26 | 4 | TG02 Compound No.14 0.25 mg/kg QWx3 IV | 13 | 0.069 | 0 |
| CT26 | 4 | TG03 Anti-PD-1 10 mg/kg BIWx3 IP | -2 | 0.792 | 3 |
| CT26 | 4 | TG04 Vehicle+ Radiation 8 Gy | 63 | 0.001 | 0 |
| CT26 | 4 | TG05 Compound No.14 0.25 mg/kg QWx3 IV + Radiation 8 Gy | 88 | <0.001 | 0 |
| CT26 | 4 | TG06 Compound No.14 0.25 mg/kg QWx3 IV +Anti-PD-1 10 mg/kg BIWx3 IP+ Radiation 8 Gy | 101 | <0.001 | 0 |

**Table 4b: Study 4 Combination Assessment**

| **Model** | Study Number | Combination group | Synergy Score % | P Value | Combination Assessment |
|---|---|---|---|---|---|
| CT26 | 4 | TG05 Compound No.14 0.25 mg/kg QWx3 IV + Radiation 8 Gy | -12 | 0.448 | Additive |
| CT26 | 4 | TG06 Compound No.14 0.25 mg/kg QWx3 IV +Anti-PD-1 10 mg/kg BIWx3 IP+ Radiation 8 Gy | -16 | 0.393 | Additive |

### Study 5:

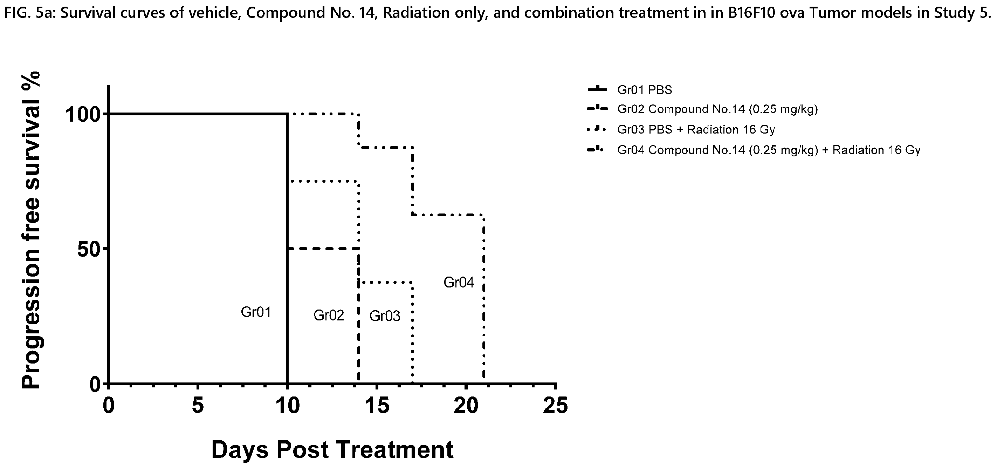

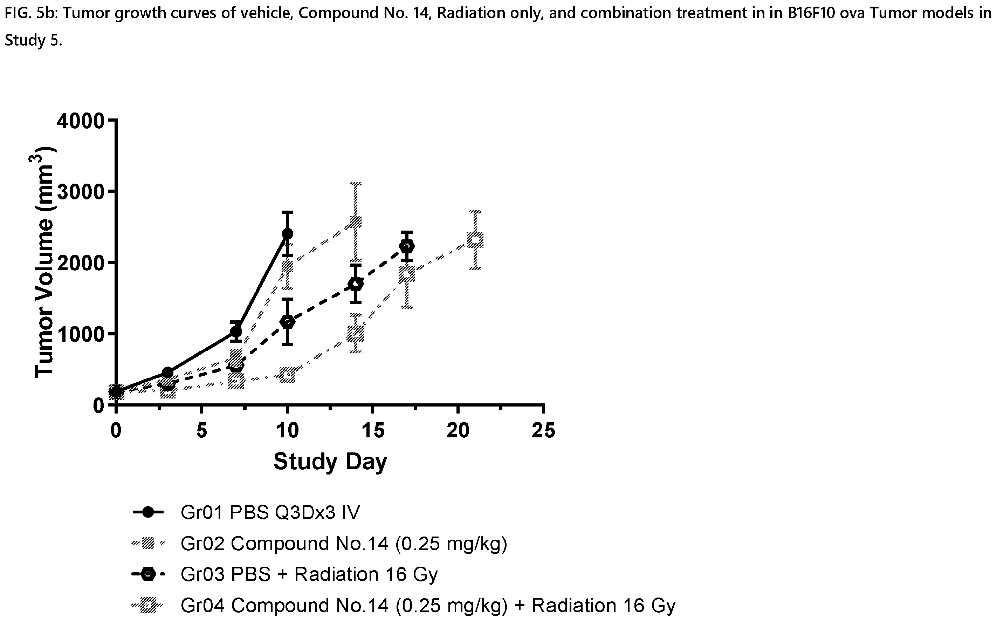

The tumor growth rate inhibition of treatment groups from Study 5 is shown in Table 5a. The combination effect for the period through Day 10 is shown in Table 5b. Kaplan-Meier survival curves are shown in Figure 5a, and tumor growth curves for each group are shown in Figure 5b.

Anti-tumor activity (when compared with vehicle) was the greatest in animals that were treated with the combination of Compound No.14, plus radiation (GRI of 67%, p value <0.001). Radiation and Compound No. 14 treatment alone also demonstrated moderate antitumor activity when compared to vehicle, although this was less robust than the double combination groups (35%, p value = 0.009, GRI 11%, p value 0.036 respectively). These treatments were all well tolerated with no animals removed from study for body weight loss greater than 20 percent.

**Table 5a: Study 5 Tumor Growth Rate Inhibition and Complete Regressions**

| **Model** | **Study Number** | **Treatment Group** | **GRI (%) (Day 10)** | **P value** | **Number of Animals Removed** |
|---|---|---|---|---|---|
| B16F10-Ova | 5 | TG01 Vehicle Q3Dx3 IV (reference group) | N/A | N/A | 8 |
| B16F10-Ova | 5 | TG02 Compound No.14 0.25 mg/kg Q3Dx3 IV | 11 | 0.036 | 4 |
| B16F10-Ova | 5 | TG03 Vehicle+ radiation 16 Gy | 35 | 0.009 | 2 |
| B16F10-Ova | 5 | TG04 Compound No.14 0.25 mg/kg Q3Dx3 IV + radiation 16 Gy | 67 | <0.001 | 0 |

**Table 5b: Study 5 Combination Assessment**

| **Model** | Study Number | Combination group | Synergy Score % | P Value | Combination Assessment |
|---|---|---|---|---|---|
| B16F10-Ova | 5 | TG04 Compound No.14 0.25 mg/kg Q3Dx3 IV + radiation 16 Gy | -21 | 0.105 | Additive |

### Example 2: Clinical Study Evaluating Compound No. 14 in Combination with an Anti-PD-1 Antibody and radiation in Treatment of Patients with Metastatic Solid Tumors

An open-label, phase 1, dose escalation study will be conducted to evaluate safety, tolerability, and preliminary anti-tumor activity of Compound No. 14 and pembrolizumab following radiation therapy in the treatment of non-small cell lung cancer (NSCLC), triple negative breast cancer (TNBC), or squamous-cell carcinoma of the head and neck (SCCHN) patients who have progressed or are progressing on checkpoint inhibitors (CPIs). The information obtained during this study will be used to estimate the maximum tolerated dose (MTD) and determine the recommended phase 2 dose (RP2D) of this combination.

Approximately 46 patients with metastatic NSCLC, TNBC, or SCCHN will be enrolled in this study, to achieve a maximum of 39 dose limiting toxicity (DLT) evaluable patients. All patients will receive 8 Gy x 3 doses (i.e., 3 fractions at 8 Gy each) of image-guided radiation therapy, followed by intravenous (IV) administration of pembrolizumab and Compound No. 14.

Pembrolizumab will be administered at 200 mg IV on Day 1 of every 21-day cycle, with a minimum for 40 hours between the last fraction of radiation therapy and the initiation of IV pembrolizumab. Compound No. 14 will be administered in a dose escalating fashion following the Bayesian Optimal INterval (BOIN) design, with an initial explorable dose range of 0.2 to 2.5 mg administered on Days 1, 8, and 15 of every 21-day cycle. Patients will only receive Compound No. 14 with pembrolizumab at dose levels that were previously deemed safe in the dose finding Phase 1 study TAK-676-1002.

Three patients will be enrolled in the initial cohort at the previously identified starting dose level of Compound No. 14. Subsequent cohorts will enroll 2-3 patients per escalation/de-escalation guidelines. Administration of pembrolizumab (every 3 weeks) and Compound No. 14 (weekly) will continue until disease progression, intolerance to pembrolizumab or Compound No. 14 (defined as the development of a treatment-emergent adverse event (TEAE) that is at least possibly related to pembrolizumab or Compound No. 14 and for which dose discontinuation is recommended), or withdrawal of consent, whichever occurs first.

Adverse events (AE) will be assessed, and laboratory values, vital signs, ECGs, and other clinically-indicated examinations will be obtained to evaluate the safety and tolerability of the study drugs in combination with radiation. Toxicity will be evaluated according to NCI CTCAE, version 5.0. Common Terminology Criteria for Adverse Events (CTCAE), Version 4.03, U.S. Department of Health and Human Services National Cancer Institute. 14 June 2010. A DLT will be defined as any of the treatment-emergent AEs (TEAEs) described in the safety evaluation that occurs during Cycle 1 and are considered by the investigator to be at least possibly related to Compound No. 14 in combination with pembrolizumab and radiation. TEAEs meeting DLT definitions occurring in later cycles will be considered in the determination of RP2D of Compound No. 14.

Radiological evaluations (CT scan and/or magnetic resonance imaging (MRI) as clinically indicated) will be employed to assess the status of the patient's underlying disease. Banked formalin fixed paraffin-embedded tumor tissue or a minimum number of unstained slides of the tumor tissue will be collected, if available, from all enrolled patients to assess baseline features such as gene mutations, gene signatures, tumor mutation burden, immune cell content, or biomarkers of response or resistance to treatment that may emerge from future nonclinical or clinical studies. All patients with a safely accessible lesion outside the radiation field and in whom a fresh tumor biopsy enrolling at dose levels of Compound No. 14 which have been shown to have pharmacodynamic activity will have mandatory tumor biopsy performed as per Schedule of Events (SOE).

Serial blood samples will be collected for circulating biomarkers (peripheral proteins, cytokines, and chemokines, including IP-10, an IFN-inducible chemokine), immunophenotyping, mRNA expression, receptor sequencing, and cell-free DNA. An evaluation of disease response will be performed using the RECIST version 1.1 (as determined by the investigator) and as per SOE. Serial blood samples for determination of the plasma concentration of Compound No. 14 and related metabolites to understand Compound No. 14 metabolism and excretion mechanisms will be obtained at prespecified time points as described in the SOE.

### Primary Endpoints

The primary endpoints for this phase 1 trial may include frequency and severity of TEAEs; number of patients with DLTs; number/percentage of patients with 1 or more treatment-emergent serious adverse events (TESAE); and number/percentage of patients with 1 or more TEAE leading to dose modifications and treatment discontinuations.

Safety endpoints will be evaluated according to the National Cancer Institute Common Terminology Criteria for Adverse Events (NCI CTCAE), Version 5.0. Common Terminology Criteria for Adverse Events (CTCAE), Version 5.0., U.S. Department of Health and Human Services, National Institutes of Health, National Cancer Institute (November 2017).

### Secondary Endpoints

The secondary endpoints for this phase 1 trial may include overall response rate (ORR) (i.e., confirmed complete response (cCR) + confirmed partial response (cPR)); abscopal response rate (ORRabscopal) (i.e., cCRabscopal + cPRabscopal of tumor lesions lying outside of the radiation field); localized response rate (ORRlocal) (i.e., cCRlocal + cPRlocal of tumor lesions lying within the radiation field); duration of response for all tumor lesions (DOR), for tumors lying within the radiation field (DORlocal), and those lying outside of the radiation field (DORabscopal); and time to response for all tumor lesions (TTR), for tumors lying within the radiation field (TTRlocalized) and those lying outside of the radiation field (TTRabscopal).

The response assessments are to be made by the investigator per Response Evaluation Criteria in Solid Tumors (RECIST) v.1.1. Eur. J. Cancer, 45(2): 228-47 (2009). To allow for instances of pseudoprogression, allowances will be made for patients to continue on treatment after an initial assessment of PD, assuming subsequent imaging does not confirm PD.

The trial will be conducted in conformance with Good Clinical Practices.

While certain embodiments have been illustrated and described, it should be understood that changes and modifications can be made therein in accordance with ordinary skill in the art without departing from the technology in its broader aspects as defined in the following claims.

The present disclosure is not to be limited in terms of the particular embodiments described in this application. Functionally equivalent methods and compositions within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, reagents, compounds compositions or biological systems, which can of course vary. It is also to be understood that the terminology used herein is for the purpose of describing particular embodiments only, and is not intended to be limiting.

## Claims

1. A compound for use in a method of treating cancer, wherein the method comprises administering the compound in combination with radiation and a checkpoint inhibitor to a patient in need of such treatment, wherein the compound is Compound No. 14, having the following structure: 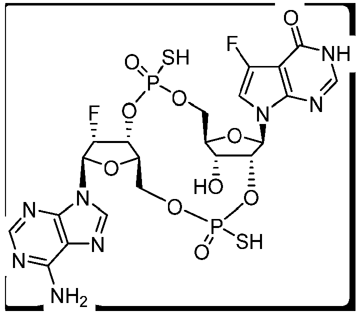
or a pharmaceutically acceptable salt thereof; and
the checkpoint inhibitor is an anti-PD-1 antibody.

2. The compound for use of claim 1, wherein the anti-PD-1 antibody is selected from the group consisting of nivolumab, pembrolizumab, lambrolizumab, pidilizumab, BMS-936559, and AMP-224.

3. The compound for use of claim 1 or 2, wherein the radiation is particle radiation and/or wherein the radiation is administered by external beam radiation.

4. The compound for use of any one of claims 1 to 3, wherein Compound No. 14, or a pharmaceutically acceptable salt thereof, is administered orally, intravenously, or by intravenous infusion.

5. The compound for use of any one of claims 1 to 4, where Compound No. 14 and the checkpoint inhibitor are administered concurrently.

6. The compound for use of any one of claims 1 to 4, where Compound No. 14 and the checkpoint inhibitor are administered sequentially in separate pharmaceutical compositions.

7. The compound for use of any one of claims 1 to 6, wherein the radiation, Compound No. 14, and checkpoint inhibitor are administered sequentially.

8. The compound for use of any one of claims 1 to 7, wherein the radiation is administered before Compound No. 14 and the checkpoint inhibitor.

9. The compound for use of any one of claims 1 to 8, wherein the cancer is melanoma, lung cancer, renal cancer, lymphoma, head and neck cancer, urothelial cancer, prostate cancer, bladder cancer, breast cancer, gastric cancer, colorectal cancer, leukemia, cervical cancer, microsatellite instability-high cancer, hepatocellular carcinoma, or Merkel cell carcinoma, preferably wherein:
(a) the melanoma is metastatic melanoma, unresectable melanoma, or cutaneous melanoma.
(b) the lung cancer is non-small cell lung cancer or small cell lung cancer, preferably wherein the non-small cell lung cancer is metastatic non-small cell lung cancer, metastatic squamous non-small cell lung cancer, or metastatic nonsquamous non-small cell lung cancer;
(c) the renal cancer is renal cell carcinoma;
(d) the lymphoma is classical Hodgkin lymphoma or primary mediastinal large B-cell lymphoma;
(e) the head and neck cancer is head and neck squamous cell carcinoma;
(f) the urothelial cancer is urothelial carcinoma;
(g) the prostate cancer is hormone-refractory prostate cancer;
(h) the gastric cancer is gastroesophageal junction adenocarcinoma;
(i) the cancer is microsatellite instability-high cancer; or
(j) the breast cancer is triple negative breast cancer.

10. The compound for use of any one of claims 1 to 8, wherein the cancer is a metastatic solid tumor.

11. The compound for use of any one of claims 1 to 10, wherein the checkpoint inhibitor is administered once every twelve weeks, once every four weeks, once every three weeks, once every two weeks, once every week, twice a week, three times a week, or daily.

12. The compound for use of any one of claims 1 to 11, wherein the checkpoint inhibitor is administered on Day 1 or Day 2 of a treatment cycle, preferably wherein the treatment cycle is 14 days, 21 days, 28 days, or 84 days.

13. The compound for use of any one of claims 1 to 10, wherein Compound No. 14 is administered on Days 1, 8, and 15 of a treatment cycle, or on Days 1, 4, 8, and 11 of a treatment cycle, or on Days 1 and 8 of a treatment cycle, preferably wherein the treatment cycle is 14 days, 21 days, 28 days, or 84 days.

14. The compound for use of any one of claims 1 to 10, wherein the radiation is administered between Day -8 and Day -1 of a treatment cycle or on Day 1 of a treatment cycle, preferably wherein the treatment cycle is 14 days, 21 days, 28 days, or 84 days.

15. The compound for use of any one of claims 1 to 10, wherein Compound No. 14 is administered on Days 1, 8, and 15 of a treatment cycle, the checkpoint inhibitor is administered on Day 1 of a treatment cycle, and the radiation is administered between Day -8 and Day -1 of a treatment cycle, preferably wherein the radiation is administered at least 40 hours before administration of the checkpoint inhibitor or Compound No. 14.

16. The compound for use of any of claims 12 to 15, wherein the treatment cycle is 21 days.

17. The compound for use of any one of claims 1 to 16, wherein:
(a) the checkpoint inhibitor is administered in an amount of 200 mg; and/or
(b) Compound No. 14 is administered in an amount of 0.05 mg, 0.1 mg, 0.2 mg, 0.4 mg, 0.8 mg, 1.2 mg, 1.6 mg, 2.0 mg, or 2.5 mg, or in an amount of from 0.05 mg to 3.5 mg, or in an amount of from 0.1 mg to 3.5 mg, or in an amount of from 0.2 mg to 2.5 mg, or in an amount of from 0.05 mg to 1.2 mg, or in an amount of from 0.1 mg to 1.2 mg, or in an amount of from 0.2 mg to 1.2 mg; and/or
(c) the radiation is administered at a fraction dose of about 5 Gy to about 20 Gy, or about 6 Gy to about 18 Gy, or about 8 Gy to about 16 Gy, preferably wherein:
(i) the radiation is administered at a fraction dose of about 5 Gy to about 10 Gy, or about 15 Gy to about 20 Gy, preferably at a fraction dose of about 8 Gy or about 16 Gy; and/or
(ii) the radiation is administered in 1 fraction, or in 2 fractions, or in 3 fractions, or in 4 fractions, or in 5 fractions, preferably wherein the radiation is administered in 1 fraction or in 3 fractions; and/or
(iii) the radiation is administered at a fraction dose of about 8 Gy for 1 fraction, or about 8 Gy for 3 fractions, or about 16 Gy for 1 fraction; and/or
(d) the checkpoint inhibitor is administered in an amount of 200 mg;
Compound No. 14 is administered in an amount of from 0.05 mg to 2.5 mg, or in an amount of from 0.2 mg to 1.2 mg; and
the radiation is administered at a fraction dose of about 8 Gy for 1 fraction, or about 8 Gy for 3 fractions, or about 16 Gy for 1 fraction.

18. A compound for use in a method of treating cancer in a patient in need thereof, wherein the patient is a patient that has undergone radiation therapy and wherein the method comprises administering the compound in combination with a checkpoint inhibitor, wherein the compound is Compound No. 14, having the following structure: 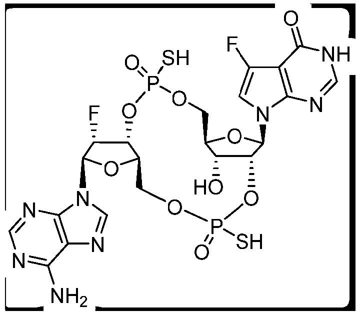
or a pharmaceutically acceptable salt thereof; and
the checkpoint inhibitor is an anti-PD-1 antibody, preferably wherein Compound No. 14 and the checkpoint inhibitor are administered to the patient from 1 day to 3 months after the patient received treatment with radiation.

## Patentansprüche

1. Verbindung zur Verwendung in einem Verfahren zum Behandeln von Krebs, wobei das Verfahren Verabreichen der Verbindung in Kombination mit Strahlung und einem Checkpoint-Inhibitor an einen Patienten, der eine derartige Behandlung benötigt, umfasst, wobei die Verbindung Verbindung Nr. 14 ist, die die folgende Struktur aufweist: 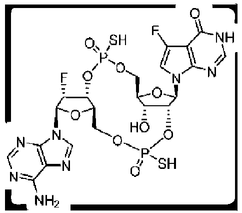
oder ein pharmazeutisch verträgliches Salz davon; und
der Checkpoint-Inhibitor ein Anti-PD-1-Antikörper ist.

2. Verbindung zur Verwendung nach Anspruch 1, wobei der Anti-PD-1-Antikörper aus der Gruppe bestehend aus Nivolumab, Pembrolizumab, Lambrolizumab, Pidilizumab, BMS-936559 und AMP-224 ausgewählt ist.

3. Verbindung zur Verwendung nach Anspruch 1 oder 2, wobei die Strahlung Teilchenstrahlung ist und/oder wobei die Strahlung durch externe Direktstrahlung verabreicht wird.

4. Verbindung zur Verwendung nach einem der Ansprüche 1 bis 3, wobei Verbindung Nr. 14 oder ein pharmazeutisch verträgliches Salz davon oral, intravenös oder durch intravenöse Infusion verabreicht wird.

5. Verbindung zur Verwendung nach einem der Ansprüche 1 bis 4, wobei Verbindung Nr. 14 und der Checkpoint-Inhibitor gleichzeitig verabreicht werden.

6. Verbindung zur Verwendung nach einem der Ansprüche 1 bis 4, wobei Verbindung Nr. 14 und der Checkpoint-Inhibitor nacheinander in getrennten pharmazeutischen Zusammensetzungen verabreicht werden.

7. Verbindung zur Verwendung nach einem der Ansprüche 1 bis 6, wobei die Strahlung, Verbindung Nr. 14 und der Checkpoint-Inhibitor nacheinander verabreicht werden.

8. Verbindung zur Verwendung nach einem der Ansprüche 1 bis 7, wobei die Strahlung vor Verbindung Nr. 14 und dem Checkpoint-Inhibitor verabreicht wird.

9. Verbindung zur Verwendung nach einem der Ansprüche 1 bis 8, wobei der Krebs ein Melanom, Lungenkrebs, Nierenkrebs, Lymphom, Kopf-Hals-Krebs, Urothelkrebs, Prostatakrebs, Blasenkrebs, Brustkrebs, Magenkrebs, Darmkrebs, Leukämie, Gebärmutterhalskrebs, Krebs mit hoher Mikrosatelliteninstabilität, Leberzellkarzinom oder Merkelzellkarzinom ist, wobei vorzugsweise:
(a) das Melanom ein metastasiertes Melanom, ein nicht resektables Melanom oder ein kutanes Melanom ist;
(b) der Lungenkrebs ein nichtkleinzelliger Lungenkrebs oder kleinzelliger Lungenkrebs ist, wobei der nichtkleinzellige Lungenkrebs vorzugsweise metastasierter nichtkleinzelliger Lungenkrebs, metastasierter nichtkleinzelliger Plattenepithel-Lungenkrebs oder metastasierter nichtkleinzelliger Nicht-Plattenepithel-Lungenkrebs ist;
(c) der Nierenkrebs ein Nierenzellkarzinom ist;
(d) das Lymphom ein klassisches Hodgkin-Lymphom oder ein primäres mediastinales großzelliges B-Zell-Lymphom ist;
(e) der Kopf-Hals-Krebs ein Plattenepithelkarzinom des Kopf-Hals-Bereichs ist;
(f) der Urothelkrebs ein Urothelkarzinom ist;
(g) der Prostatakrebs hormonrefraktärer Prostatakrebs ist;
(h) der Magenkrebs ein Adenokarzinom des gastroösophagealen Übergangs ist;
(i) der Krebs ein Krebs mit hoher Mikrosatelliteninstabilität ist; oder
(j) der Brustkrebs triple-negativer Brustkrebs ist.

10. Verbindung zur Verwendung nach einem der Ansprüche 1 bis 8, wobei der Krebs ein metastasierter solider Tumor ist.

11. Verbindung zur Verwendung nach einem der Ansprüche 1 bis 10, wobei der Checkpoint-Inhibitor einmal pro zwölf Wochen, einmal pro vier Wochen, einmal pro drei Wochen, einmal pro zwei Wochen, einmal pro Woche, zweimal pro Woche, dreimal pro Woche oder täglich verabreicht wird.

12. Verbindung zur Verwendung nach einem der Ansprüche 1 bis 11, wobei der Checkpoint-Inhibitor an Tag 1 oder Tag 2 eines Behandlungszyklus verabreicht wird, wobei der Behandlungszyklus vorzugsweise 14 Tage, 21 Tage, 28 Tage oder 84 Tage ist.

13. Verbindung zur Verwendung nach einem der Ansprüche 1 bis 10, wobei Verbindung Nr. 14 an Tag 1, 8 und 15 eines Behandlungszyklus oder an Tag 1, 4, 8 und 11 eines Behandlungszyklus oder an Tag 1 und 8 eines Behandlungszyklus verabreicht wird, wobei der Behandlungszyklus vorzugsweise 14 Tage, 21 Tage, 28 Tage oder 84 Tage ist.

14. Verbindung zur Verwendung nach einem der Ansprüche 1 bis 10, wobei die Strahlung zwischen Tag -8 und Tag -1 eines Behandlungszyklus oder an Tag 1 eines Behandlungszyklus verabreicht wird, wobei der Behandlungszyklus vorzugsweise 14 Tage, 21 Tage, 28 Tage oder 84 Tage ist.

15. Verbindung zur Verwendung nach einem der Ansprüche 1 bis 10, wobei Verbindung Nr. 14 an Tag 1, 8 und 15 eines Behandlungszyklus verabreicht wird, der Checkpoint-Inhibitor an Tag 1 eines Behandlungszyklus verabreicht wird und die Strahlung zwischen Tag -8 und Tag -1 eines Behandlungszyklus verabreicht wird, wobei die Strahlung vorzugsweise mindestens 40 Stunden vor Verabreichung des Checkpoint-Inhibitors oder Verbindung Nr. 14 verabreicht wird.

16. Verbindung zur Verwendung nach einem der Ansprüche 12 bis 15, wobei der Behandlungszyklus 21 Tage ist.

17. Verbindung zur Verwendung nach einem der Ansprüche 1 bis 16, wobei:
(a) der Checkpoint-Inhibitor in einer Menge von 200 mg verabreicht wird; und/oder
(b) Verbindung Nr. 14 in einer Menge von 0,05 mg, 0,1 mg, 0,2 mg, 0,4 mg, 0,8 mg, 1,2 mg, 1,6 mg, 2,0 mg oder 2,5 mg oder in einer Menge von 0,05 mg bis 3,5 mg oder in einer Menge von 0,1 mg bis 3,5 mg oder in einer Menge von 0,2 mg bis 2,5 mg oder in einer Menge von 0,05 mg bis 1,2 mg oder in einer Menge von 0,1 mg bis 1,2 mg oder in einer Menge von 0,2 mg bis 1,2 mg verabreicht wird; und/oder
(c) die Strahlung in einer Fraktionsdosis von etwa 5 Gy bis etwa 20 Gy oder von etwa 6 Gy bis etwa 18 Gy oder von etwa 8 Gy bis etwa 16 Gy verabreicht wird, wobei vorzugsweise:
(i) die Strahlung in einer Fraktionsdosis von etwa 5 Gy bis etwa 10 Gy oder von etwa 15 Gy bis etwa 20 Gy, vorzugsweise in einer Fraktionsdosis von etwa 8 Gy bis etwa 16 Gy verabreicht wird; und/oder
(ii) die Strahlung in 1 Fraktion oder in 2 Fraktionen oder in 3 Fraktionen oder in 4 Fraktionen oder in 5 Fraktionen verabreicht wird, wobei die Strahlung in vorzugsweise in 1 Fraktion oder in 3 Fraktionen verabreicht wird; und/oder
(iii) die Strahlung in einer Fraktionsdosis von etwa 8 Gy in 1 Fraktion oder etwa 8 Gy in 3 Fraktionen oder etwa 16 Gy in 1 Fraktion verabreicht wird; und/oder
(d) der Checkpoint-Inhibitor in einer Menge von 200 mg verabreicht wird;
Verbindung Nr. 14 in einer Menge von 0,05 mg bis 2,5 mg oder in einer Menge von 0,2 mg bis 1,2 mg verabreicht wird; und
die Strahlung in einer Fraktionsdosis von etwa 8 Gy in 1 Fraktion oder etwa 8 Gy in 3 Fraktionen oder etwa 16 Gy in 1 Fraktion verabreicht wird.

18. Verbindung zur Verwendung in einem Verfahren zum Behandeln von Krebs bei einem Patienten, der dies benötigt, wobei der Patient ein Patient ist, der sich einer Strahlenbehandlung unterzogen hat und wobei das Verfahren Verabreichen der Verbindung in Kombination mit einem Checkpoint-Inhibitor umfasst, wobei die Verbindung Verbindung Nr. 14 ist, die die folgender Struktur aufweist: 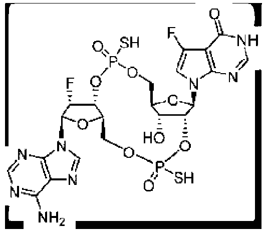
oder ein pharmazeutisch verträgliches Salz davon; und
der Checkpoint-Inhibitor ein Anti-PD-1-Antikörper ist, wobei Verbindung Nr. 14 und der Checkpoint-Inhibitor dem Patienten vorzugsweise von Tag 1 bis 3 Monate, nachdem der Patient eine Behandlung mit Strahlung erhalten hat, verabreicht werden.

## Revendications

1. Composé pour une utilisation dans un procédé de traitement du cancer, dans lequel le procédé comprend l'administration du composé en combinaison avec un rayonnement et un inhibiteur de point de contrôle à un patient qui a besoin d'un tel traitement, dans lequel le composé est le composé n° 14, ayant la structure suivante : 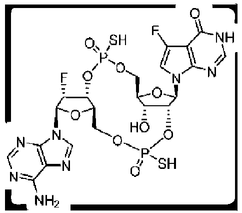
ou un sel pharmaceutiquement acceptable de celui-ci ; et
l'inhibiteur de point de contrôle est un anticorps anti-PD-1.

2. Composé pour une utilisation selon la revendication 1, dans lequel l'anticorps anti-PD-1 est choisi dans le groupe constitué du nivolumab, du pembrolizumab, du lambrolizumab, du pidilizumab, du BMS-936559, et de l'AMP-224.

3. Composé pour une utilisation selon la revendication 1 ou 2, dans lequel le rayonnement est un rayonnement de particules et/ou dans lequel le rayonnement est administré par rayonnement à faisceau externe.

4. Composé pour une utilisation selon l'une quelconque des revendications 1 à 3, dans lequel le composé n° 14, ou un sel pharmaceutiquement acceptable de celui-ci, est administré par voie orale, intraveineuse, ou par perfusion intraveineuse.

5. Composé pour une utilisation selon l'une quelconque des revendications 1 à 4, où le composé n° 14 et l'inhibiteur de point de contrôle sont administrés simultanément.

6. Composé pour une utilisation selon l'une quelconque des revendications 1 à 4, où le composé n° 14 et l'inhibiteur de point de contrôle sont administrés de manière séquentielle dans des compositions pharmaceutiques distinctes.

7. Composé pour une utilisation selon l'une quelconque des revendications 1 à 6, dans lequel le rayonnement, le composé n° 14, et l'inhibiteur de point de contrôle sont administrés de manière séquentielle.

8. Composé pour une utilisation selon l'une quelconque des revendications 1 à 7, dans lequel le rayonnement est administré avant le composé n° 14 et l'inhibiteur de point de contrôle.

9. Composé pour une utilisation selon l'une quelconque des revendications 1 à 8, dans lequel le cancer est un mélanome, un cancer du poumon, un cancer du rein, un lymphome, un cancer des voies aérodigestives supérieures, un cancer urothélial, un cancer de la prostate, un cancer de la vessie, un cancer du sein, un cancer de l'estomac, un cancer colorectal, une leucémie, un cancer du col de l'utérus, un cancer à instabilité microsatellitaire élevée, un carcinome hépatocellulaire, ou un carcinome à cellules de Merkel, de préférence dans lequel :
(a) le mélanome est un mélanome métastatique, un mélanome non résécable, ou un mélanome cutané.
(b) le cancer du poumon est un cancer du poumon non à petites cellules ou un cancer du poumon à petites cellules, de préférence dans lequel le cancer du poumon non à petites cellules est un cancer du poumon non à petites cellules métastatique, un cancer du poumon non à petites cellules squameux métastatique, ou un cancer du poumon non à petites cellules non squameux métastatique ;
(c) le cancer du rein est un carcinome à cellules rénales ;
(d) le lymphome est un lymphome de Hodgkin classique ou un lymphome médiastinal primitif à grandes cellules B ;
(e) le cancer des voies aérodigestives supérieures est un carcinome à cellules squameuses des voies aérodigestives supérieures ;
(f) le cancer urothélial est un carcinome urothélial ;
(g) le cancer de la prostate est un cancer de la prostate hormono-résistant ;
(h) le cancer de l'estomac est un adénocarcinome de la jonction gastro-œsophagienne ;
(i) le cancer est un cancer à instabilité microsatellitaire élevée ; ou
(j) le cancer du sein est un cancer du sein triple négatif.

10. Composé pour une utilisation selon l'une quelconque des revendications 1 à 8, dans lequel le cancer est une tumeur solide métastatique.

11. Composé pour une utilisation selon l'une quelconque des revendications 1 à 10, dans lequel l'inhibiteur de point de contrôle est administré une fois toutes les douze semaines, une fois toutes les quatre semaines, une fois toutes les trois semaines, une fois toutes les deux semaines, une fois par semaine, deux fois par semaine, trois fois par semaine, ou quotidiennement.

12. Composé pour une utilisation selon l'une quelconque des revendications 1 à 11, dans lequel l'inhibiteur de point de contrôle est administré le jour 1 ou le jour 2 d'un cycle de traitement, de préférence dans lequel le cycle de traitement est de 14 jours, 21 jours, 28 jours, ou 84 jours.

13. Composé pour une utilisation selon l'une quelconque des revendications 1 à 10, dans lequel le composé n° 14 est administré les jours 1, 8, et 15 d'un cycle de traitement, ou les jours 1, 4, 8, et 11 d'un cycle de traitement, ou les jours 1 et 8 d'un cycle de traitement, de préférence dans lequel le cycle de traitement est de 14 jours, 21 jours, 28 jours, ou 84 jours.

14. Composé pour une utilisation selon l'une quelconque des revendications 1 à 10, dans lequel le rayonnement est administré entre le jour -8 et le jour -1 d'un cycle de traitement ou le jour 1 d'un cycle de traitement, de préférence dans lequel le cycle de traitement est de 14 jours, 21 jours, 28 jours, ou 84 jours.

15. Composé pour une utilisation selon l'une quelconque des revendications 1 à 10, dans lequel le composé n° 14 est administré les jours 1, 8 et 15 d'un cycle de traitement, l'inhibiteur de point de contrôle est administré le jour 1 d'un cycle de traitement, et le rayonnement est administré entre le jour -8 et le jour -1 d'un cycle de traitement, de préférence dans lequel le rayonnement est administré au moins 40 heures avant l'administration de l'inhibiteur de point de contrôle ou du composé n° 14.

16. Composé pour une utilisation selon l'une quelconque des revendications 12 à 15, dans lequel le cycle de traitement est de 21 jours.

17. Composé pour une utilisation selon l'une quelconque des revendications 1 à 16, dans lequel :
(a) l'inhibiteur de point de contrôle est administré en une quantité de 200 mg ; et/ou
(b) le composé n° 14 est administré en une quantité de 0,05 mg, 0,1 mg, 0,2 mg, 0,4 mg, 0,8 mg, 1,2 mg, 1,6 mg, 2,0 mg, ou 2,5 mg, ou en une quantité comprise entre 0,05 mg et 3,5 mg, ou en une quantité comprise entre 0,1 mg et 3,5 mg, ou en une quantité comprise entre 0,2 mg et 2,5 mg, ou en une quantité comprise entre 0,05 mg et 1,2 mg, ou en une quantité comprise entre 0,1 mg et 1,2 mg, ou en une quantité comprise entre 0,2 mg et 1,2 mg ; et/ou
(c) le rayonnement est administré à une dose fractionnée d'environ 5 Gy à environ 20 Gy, ou d'environ 6 Gy à environ 18 Gy, ou d'environ 8 Gy à environ 16 Gy, de préférence dans lequel :
(i) le rayonnement est administré à une dose fractionnée d'environ 5 Gy à environ 10 Gy, ou d'environ 15 Gy à environ 20 Gy, de préférence à une dose fractionnée d'environ 8 Gy ou d'environ 16 Gy ; et/ou
(ii) le rayonnement est administré en 1 fraction, ou en 2 fractions, ou en 3 fractions, ou en 4 fractions, ou en 5 fractions, de préférence dans lequel le rayonnement est administré en 1 fraction ou en 3 fractions ; et/ou
(iii) le rayonnement est administré à une dose fractionnée d'environ 8 Gy pour 1 fraction, ou d'environ 8 Gy pour 3 fractions, ou d'environ 16 Gy pour 1 fraction ; et/ou
(d) l'inhibiteur de point de contrôle est administré en une quantité de 200 mg ;
le composé n° 14 est administré en une quantité comprise entre 0,05 mg et 2,5 mg, ou en une quantité comprise entre 0,2 mg et 1,2 mg ; et
le rayonnement est administré à une dose fractionnée d'environ 8 Gy pour 1 fraction, ou d'environ 8 Gy pour 3 fractions, ou d'environ 16 Gy pour 1 fraction.

18. Composé pour une utilisation dans un procédé de traitement du cancer chez un patient qui en a besoin, dans lequel le patient est un patient qui a subi une radiothérapie et dans lequel le procédé comprend l'administration du composé en combinaison avec un inhibiteur de point de contrôle, dans lequel le composé est le composé n° 14, ayant la structure suivante : 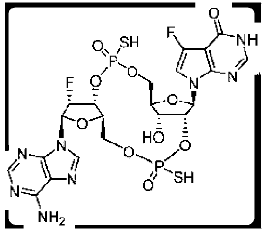
ou un sel pharmaceutiquement acceptable de celui-ci ; et
l'inhibiteur de point de contrôle est un anticorps anti-PD-1, de préférence dans lequel le composé n° 14 et l'inhibiteur de point de contrôle sont administrés au patient de 1 jour à 3 mois après que le patient a reçu un traitement par radiothérapie.
